# EUROPEAN PATENT SPECIFICATION

(11) **EP 1 451 290 B1**
(45) Date of publication and mention of the grant of the patent: **05.01.2011**
(21) Application number: 02797640.6
(22) Date of filing: 29.08.2002
(51) Int. Cl.: C12M 3/00

(54) **A UNIT AND A PROCESS FOR CARRYING OUT HIGH CELL DENSITY FERMENTATION**
VORRICHTUNG UND VERFAHREN ZUR FERMENTATION IN HOHEN ZELLDICHTEN
UNITE ET PROCEDE DE FERMENTATION A HAUTE DENSITE CELLULAIRE

(30) Priority: 31.08.2001 US 316837 P
(43) Date of publication of application: 01.09.2004
(73) Proprietor: Bayer Schering Pharma Aktiengesellschaft, 13353 Berlin (DE)
(72) Inventor: HENZLER, Hans-Jürgen, 42655 Solingen (DE); KAULING, Jörg, 51069 Köln (DE); SCHMITT, Franz, 51465 Bergisch Gladbach (DE); BECKERS, Erhard, 51399 Burscheid (DE); BÖDEKER, Berthold, 42113 Wuppertal (DE); VON HUGO, Hasso, 42115 Wuppertal (DE); KONSTANTINOV, Konstantin, Walnut Creek, CA 94596 (US); NAVEH, David, Piedmont, CA 94611 (US); STEINER, Ulrich, 40699 Erkrath (DE)
(86) International application number: PCT/EP2002/009620
(87) International publication number: WO 2003/020919

(56) References cited:
- US-A- 5 733 776
- US-A- 5 817 505

## Description

The present invention relates to a continuous process for cultivating suspended animal or plant cell lines with the aim of efficiently producing biological products. The invention also relates to units and apparatuses in which the process according to the invention for cultivating suspended animal or plant cell lines can be carried out.

Cell cultures are highly important for the production of biologically active substances and pharmaceutically active products. In particular the cultivation of the cells, which are used frequently and are freely suspended in the nutrient medium, is difficult and complicated since, in contrast to microorganisms or adherent cells, they are very sensitive to mechanical stress and an insufficient supply of substrate. For this reason the units and apparatuses according to the invention and the technical process used are crucially important for an effective method of production.

In the majority of technical processes for cultivating suspended animal or plant cell lines batch processes are used. Such processes have the disadvantage that the cell count and the concentrations of the nutrient medium and the metabolites change continuously over the batch cycle of days or weeks and that dead cells accumulate in the later phase of the fermentation and the products formed undergo enzymatic or spontaneous degradation. Thus continuous fermentation processes are recommendable particularly for the production of instable active compounds.

Continuous processes are economical and competitive if high cell densities can be achieved in the fermenter and correspondingly high productivity is obtained. This requires
(1) a sufficient supply of oxygen in the fermenter to cover the high oxygen demand of the cells at high cell densities,
(2) a cell retention system allowing the effective retention of the cells in the reactor system,
(3) more reliable long-term operation with regard to the stationary operating conditions (cell, substrate, metabolite and product concentrations) and long-term sterility of the entire reactor system and
(4) a robust, simple and easily manageable process.

The process must also take into account the high sensitivity of the cells towards mechanical stress and an insufficient supply of substrate and the instability of the products.

The prior art describes a large number of apparatuses, units and processes for cultivating cell lines. The following variants of apparatuses and units are already known:

### 1. Fermenters

The bubble-free supply of oxygen via porous or diffusion membranes is the method of oxygen supply frequently selected for cell culture fermenters since the formation, ascent and bursting of bubbles on the liquid surface subjects the cells to high degrees of stress. The stirrers recommended for this purpose are relatively small, high-speed, axially transporting stirrers which are arranged centrally in membrane stators (e.g., Fenge, Fraune, Maier, 1992. BioTec, 4: 52-54). Such a reactor design is disadvantageous, both due to the high-speed, axially transporting stirrers which cause very high degrees of stress and due to the fact that a relatively low speed and a correspondingly low rate of oxygen transport takes place on the membranes located between the vessel wall and the stirrer.

A reactor design is more suitable in which the oxygen transport is intensified by large stirrers arranged at a slight distance from and over the entire height of the membranes, although due to the baffles used in this reactor design only a relatively small membrane stator and a correspondingly small mass transfer surface can be used.

Since when scaling up the process the ratio between the membrane surface area and the reactor volume changes in a manner inversely proportional to the reactor diameter, the abovementioned method of oxygen supply is only suitable for small reactors or lower cell densities.

Also, the supply of oxygen by means of large-bubble aeration and the dispersion of bubbles by means of stirring limits the cell density and the viability of the cell culture due to the large degree of mechanical stress involved.

### 2. Cell retention

In the past a number of different cell retention systems have been proposed for continuous fermentation processes, which are appropriately arranged outside the fermenter in order to allow flexible handling.

In order to minimize the damage to the cells occurring when using external apparatuses in particular as a result of the insufficient supply of oxygen to the cells and the insufficient removal of CO₂ outside the fermenter, cell retention systems with small working volumes and a correspondingly short residence time of the cells in the cell retention system are particularly desirable.

In addition to membrane filters and cross-flow filtration units with stationary and moving membranes, special centrifuges and sedimentation apparatuses have been used.

Where cell retention takes place by means of membrane filters, fouling effects are however observed which render impossible the robust low-maintenance long-term operation thereof. A reduction in fouling can be obtained by a high rate of flow on the membranes. Since high speeds in the pumps, pipelines and channels of the membrane units do however produce increased stress the need for high speeds is defeated by the requirement for low-shear treatment of the cells.

For the removal of cells by means of centrifugation special centrifuges have been developed which have the disadvantage of subjecting the cells to increased mechanical stress, since accelerations of more than two hundred times that of gravitational acceleration are used for their removal. In addition, centrifuges do not operate reliably over several weeks or months without maintenance and they also cause increased operating costs.

An additional method of removing cells from cell culture supernatants is the use of gravitational sedimentation units. The gravitational sedimentation units predominantly used in cell culture are sedimentation tanks and slanting channel systems. Compared with simple sedimentation vessels, slanting channel systems have the advantage of a considerably smaller volume.

Systems described so far (J.Stevens,u.a.: Preprint Esact-Meeting 1993 Würzburg; K.J.Thompson, J.S.Wilson: Preprint Esact-Meeting 1993 Würzburg; J.A.Searles, u.a. Biotechnol. Prog. 1994,10, 188-206; WO 94/26384) are counter current systems with a very small settling areas (Aₜₕ=z b₁ L cosα < 0.2 m²; z: number of plates; b₁: width; L: length of channels; α: inclination to the horizontal) and can therefore not be used for the production scale. US 5 817 505 describes the use of a sedimentation separator to separate hybridoma cells from antibody containing medium.

Scaling-up is a problem in counter current slanting channel systems, since the volume of the required concentrate and clear phase collection chambers of the sedimentation separator, V_{SF}, increases overproportionally as the fermenter volume V increases (V_{SF} ∝ V^{1.5} at constant perfusion rate) and increases even more with increasing perfusion rate q/V (V_{SF} ∝ (qN)^{2,15} at constant fermenter volume). The geometry of most of the slanting channel systems proposed for the cultivation of cells does however prevent their use on a large scale due to the not advantageous geometry (inflow and outflow sections and channel length) and large working volumes which result. The concentrate and clear phase collection chambers and the in- and outflowing stream channels incorporated therein are designed disadvantageously in the variants proposed. The channel lengths of the slanting channel systems used, which are in the range from 100 to 300mm, are comparably short. The most frequently proposed channel length are only 100mm. The features of the variants proposed did not however prove negative for those customers known to have used such systems since only tests on a small scale (with a fermenter volume of 1 to 25 1) were carried out.

For high cell density fermentation using cell concentrations of more than 1.5 x 10⁷ living cells per ml of reactor volume a sedimentation separator with a volume of 70 to 550 or 50 to 500 l would be necessary for a fermenter volume of 100 to 200 l (even when using the relatively quickly sedimenting BHK cells) if the a conventional settler design is used. The desired cell density of 1.5 x 10⁷ living cells per ml of reactor volume could not be obtained in such units over long periods of time, since the preferred growth rate of µ ≈ 0.4/d could not be maintained due to the long residence time in the sedimentation separator and the corresponding insufficient supply of oxygen.

Although Bayer AG's bulletin (1992, Chemie-Technik, 21(3), 118) contains a reference to long slanting channel systems of 0.2 to 2.5 m, this paper describes liquid distribution systems and concentrate collection chambers which do not satisfy the requirements concerning a small working volume. Due to the fact that in the disclosed units the medium is injected into a cup-like device (in order to reduce turbulence in the receiving chamber (32)) the volume of the receiving chamber (32) itself must be relatively large. In fact, it is impossible to construct such receiving chambers with a cup-like device and having a purely conical or pyramidal geometry. In order to place the cup-like device into the receiving chamber, the receiving chamber must have a cylindrical section in addition to a conical or pyramidal section, hence, the volume of the receiving chamber is increased. In the abovementioned example the working volumes of the sedimentation separator would be V_{S} = 50 to 100 l which is much higher than the volume of separators of the invention.

### 2.1 Cooling

In order to reduce the metabolic activity and cell deposits in the sedimentation separator the cooling of the cell culture broth in the sedimentation separator has been proposed. Due to the formation of temperature gradients (and corresponding density gradients) in the interior of the sedimentation separator this fundamentally correct proposal does however lead to convection currents. These in turn have a negative effect on the effectiveness of the cell separation. This is particularly crucial where separators with a relatively low ratio of separator surface area to separator volume are used, since in such units only relatively small volumetric throughputs per separator volume can be obtained.

### 2.2 Vibration

In order to reduce the residence time of the cells in sedimentation separators a non-defined vibration of the slanting channel system has been proposed (Bayer AG, 1992, Chemie-Technik, 21(3): 118 ; Searles, et al. 1994. Biotechnology Progress, 10: 188-206).

### 3. An inoculation fermenter

For the efficient operation of fermentation processes a specific starting cell density is required in the fermenters, since insufficient starting cell densities lead to delayed growth of the cells due to a lack of allomones. (In the case of animal cells the starting cell density should be approx. 10⁶ cells per ml.) Thus, depending on the size of the production fermenter, several pre-fermenters are necessary. For the cultivation of the cells discontinuously operated fermenters with which cell densities of 5 x 10⁶ to 8 x 10⁶ cells per ml can be obtained are usually employed due to their simpler mode of operation. This means that, for example for inoculating a 200 l production fermenter starting from cell conserve a conventional seed-train expansion with many T-flasks and 60 roller bottles would be necessary.

### 4. Low-shear pumps and pipelines

The operation of the process requires pumps and pipelines which interconnect the storage tank, the fermenter, the external sedimentation separator and the harvest vessel. In the known literature no details are given of the selection and design of the pumps and pipelines. This aspect is however highly important for long-term cultivation under sterile conditions at high cell concentrations and vitality. Attention must also be paid to the correct arrangement of the pumps in the unit as a whole. The arrangement of pumps in the concentrate recycling stream leading to the fermenter, as described in patent WO94/26384, is disadvantageous since as a result the concentrated cell suspension (i.e. a large number of cells) is exposed to the very high mechanical stress in the pumps.

Based on the above considerations the technical problem arose of developing units, apparatuses and an efficient process for fermenting shear-sensitive cells with which biological products can be produced economically and with high quality.

This problem is solved according to the invention by a fermentation unit consisting of at least one storage tank containing the nutrient medium, pumps and pipelines, a production fermenter, at least two throughflow heat exchangers and a cell retention sedimentation separator, which is optionally equipped with a vibrator (5). A continuous fermentation process with high perfusion rates is also advantageous for solving the technical problem concerned.

### Brief description of the invention

The process set-up consists of a number of apparatuses, and at least a storage tank (1) containing the nutrient medium, pumps and pipelines (7, 8), the production fermenter (2) which is at least intermittently continuously perfused, at least two throughflow heat exchangers (3) and a cell retention sedimentation separator (4) which is optionally equipped with a vibrator, the components of the unit being designed in such a manner that long-term cultivation in which the cultivated cells are subjected to low degrees of mechanical stress can be carried out over process times of longer than one month. With the aid of the units according to the invention cultivations having long-term stability over 3-5 months at cell densities of more than 1.5 x 10⁷ living cells per ml reactor volume and a viability of higher than 80% prefered 90% are obtained. In the present context viability is defined as the relative percentage of the total number of cells in the culture which consist of living cells.

The cell densities, viabilities and cultivation times can however be lower/shorter depending on the organism and type of fermentation procedure used.

In the process according to the invention it is also possible to use a specially designed and batchwise and/or continuously operated inoculation fermenter (9) whose maximum filling volume is smaller than 6% of the production fermenter volume and which nevertheless allows a 50 to 150 fold increase in cell count.

With the aid of the present invention high productivity of the process is obtained despite the use of sensitive cells and products prone to degradation.

### Detailed description of the invention

The invention relates to a fermentation unit which differs from the prior art in advantageous variants at various points of the unit. Whereas the advantageous variants can also display their advantageous effects in isolation a preferred variant is one in which several or all of the advantageous features of the unit according to the invention interact. Thus the increased oxygen transfer capacity of the main fermenter can for example be particularly effective since a higher cell density is established in the culture due to the more effective cell recycling in the sedimentation separator. Similar synergistic effects for increasing the overall productivity of the fermentation process are also obtained by the interaction of the other components of the unit according to the invention.

The invention also relates to an advantageous process for carrying out high cell density fermentation processes in units according to the invention. The process according to the invention makes it possible to use effectively the positive effect of the variants of the fermentation unit according to the invention.

In the following the advantageous variants of the fermentation unit are described:

### 1. The production fermenter

Due to its optimum design with regard to stirring the production fermenter (2) is distinguished by a high rate of mass transfer at the gas/liquid phase interface and minimum shearing stress.

Three varying fermenter types producing the abovementioned effects have been developed.

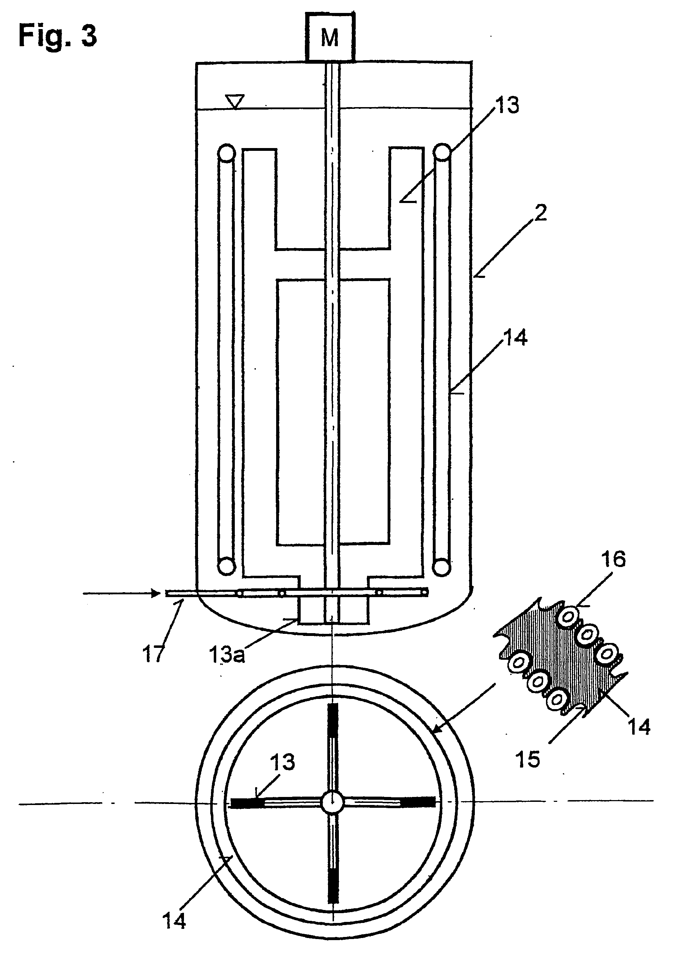

In the case of fermenter type A (Fig.3) the oxygen supply takes place via diffusion membranes. The silicon tube membranes employed which have small wall thicknesses are wound axially onto a tube stator surrounding the stirrer concentrically (14). It is advantageous for the fermenter and the tube stator to be designed in a slender fashion since this enables larger volume-specific mass transfer surfaces (A/V [m⁻¹]) to be obtained. The stirrer is a large-area, multiblade anchor stirrer (13) which is only a small distance, preferably 5 to 15 mm, away from the silicon tubes and whose stirrer blades extend over the entire length of the tube stator. Even at low stirrer rotation speeds and stirrer powers of less than 10 or 20 watts per m³ of fermenter volume this design type produces slight vibration of the silicon tubes so that an additional intensification of the mass transfer and the simultaneous cleansing of the membranes takes place during operation.

For the satisfactory suspension of the cells the stirrers employed are modified in such a manner according to the invention that they can extend to the region close to the base of the vessel. This is made possible by the tapering of the stirrer blades in the region close to the base (13a).

The fermenter according to type A does not contain any baffles. As a result the tube stator can be very large and specific mass transfer surface areas of A/V > 10/D (A: mass transfer area; V: fermenter volume; D: vessel diameter) are possible. Thus particularly large stirrers with d/D > 0.6 (d: external diameter of the stirrer) can be used, which produce a particularly low degree of shear.

The peripheral movement still prevailing in contrast to systems containing baffles surprisingly produces an additional intensification of the mass transfer and additional aeration with bubbles. The gas bubbles undergo tangential movement which at a given bubble size produces a larger content of gas in the fermenter and thus a larger phase interface.

In addition to the silicone tube stator an aeration ring (17) can be additionally incorporated at the base of the fermenter for the additional bubble aeration. This allows an additional transport of oxygen and the possibly required intensification of the removal of CO₂.

Where pure oxygen and excess pressures are used in the silicone tubes very high cell densities of up to 2 x 10⁷ living cells per ml can be obtained with fermenter type A.

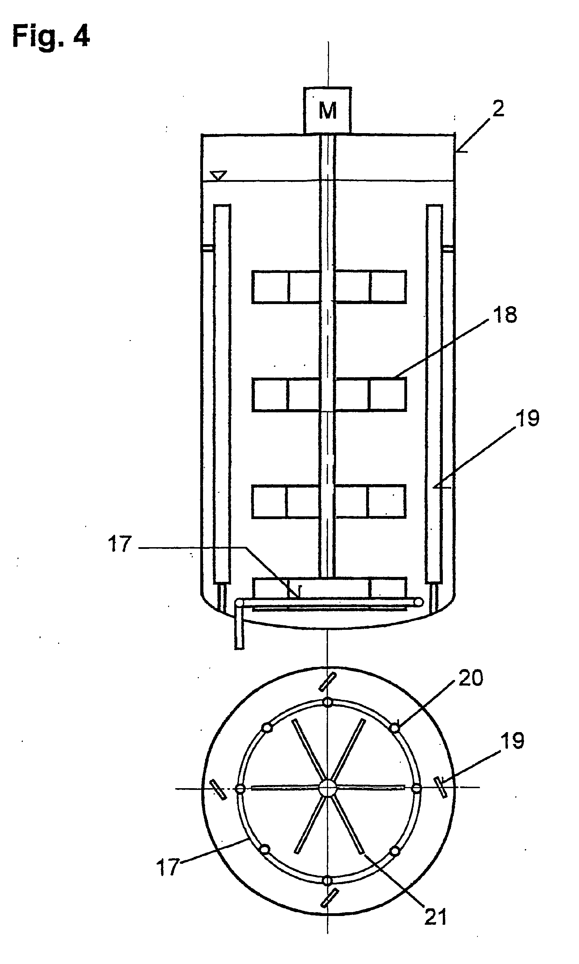

In the case of fermenter type B (Fig.4) the oxygen supply takes place solely via fine bubble aeration with oxygen.

Fine bubble aeration is obtained by means of special sintered bodies, filter plates, ceramic membranes or laser-perforated plates (20) having pores or holes of a very small size of d_{L}<15 µm. Due to the low superficial gas velocities of v < 0.5 m/h which can be used very small gas bubbles are formed which display only a slight tendency to coalesce during moderate stirring with low-speed stirrers. The oxygen demand of high cell density fermentation can thus be obtained with less than 1/10th of the superficial gas velocities which are necessary for large-bubble aeration via mechanically perforated holes (d_{L} > 0.2 mm), so that the growth of the cells is not adversely affected by the aeration process.

In addition, according to the invention large low-speed multistage blade stirrers (18) having a ratio of external stirrer diameter to internal vessel diameter of d/D > 0.5, preferably d/D > 0.6, are used for stirring, since it has surprisingly been found that high-speed, and in particular axially transporting high-speed stirrers, cause coalescence of the gas bubbles. These large stirrers produce non-coalescent low-shear distribution of the fine gas bubbles. At the same time the stirrers extending close to the base and the baffles at a distance from the vessel base produce the suspension and uniform distribution of the biomass even at very low stirrer powers of P/V < 5 W/m³ due to the tangential motion produced at the base.

The baffles (19) are not incorporated in a radial but in an inclined fashion, as a result of which a 40% higher mass transfer efficiency (mass flow per volume-specific stirrer power) and a corresponding additional reduction in the stress on the cells is obtained.

Since the baffles (19) are not located on the wall and are not only at a distance from the base but also do not extend to the surface of the liquid and are therefore covered at the top by the liquid, deposits can be substantially avoided. This creates an important prerequisite for in situ (CIP: "cleaning-in-place") cleansing.

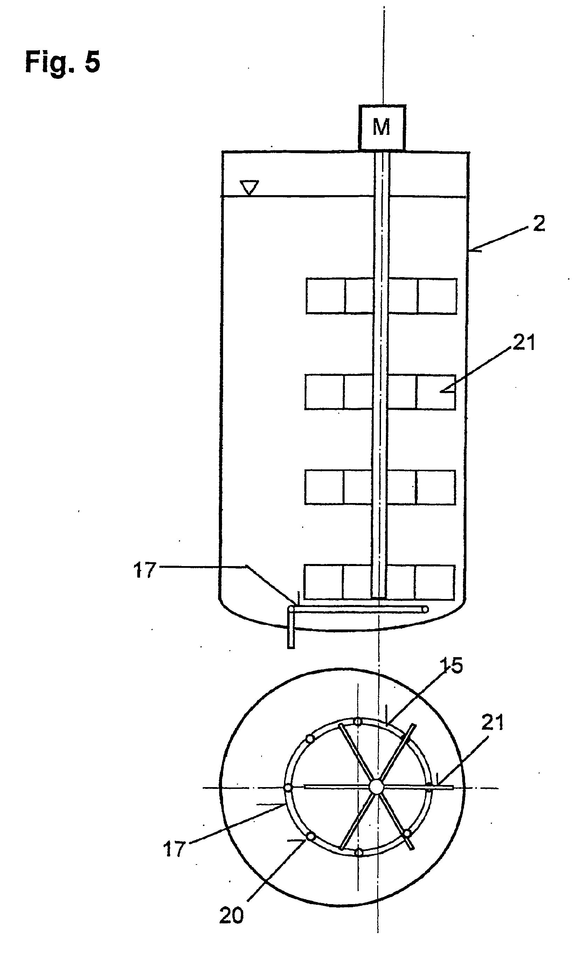

In the case of fermenter type C (Fig.5) the oxygen supply takes place (as in the case of B) via fine bubble oxygen aeration. In contrast to B a baffle-free reactor with an eccentrically arranged multistage blade stirrer (21) is used.

The eccentric arrangement of the stirrer (21) intensifies axial mixing but does not however completely suppress the peripheral flow component, as is the case when using baffles, and in particular wall baffles. As already mentioned above the peripheral movement still prevailing surprisingly intensifies the mass transfer at the gas/liquid phase interface. The gas bubbles undergo tangential movement which at a given bubble size produces a higher gas content and a correspondingly larger phase interface.

The eccentric arrangement of the stirrer according to the invention substantially prevents cell deposits and creates an ideal prerequisite for CIP cleansing of the fermenters.

According to the invention, when using pure oxygen cell densities of up to 5 x 10⁷ living cells per ml can be achieved with fermenter types B and C at viabilities of > 90%.

### 2. The inoculation fermenter

In order to simplify the fermentation process the quantity of seed for the production fermenter is produced in a single-stage pre-culture. This is inoculated directly using a stock culture tube without any additional cultivation step. In order to obtain the required 50- to 150-fold increase in cell count in the inoculation fermenter (9) a special type of fermenter geometry and a special type of pre-culture is however required. In particular it is necessary for the culture to be inoculated at a small working volume which is increased in size during the pre-culture step by the addition of nutrient medium.

The special fermenter geometry was necessary since a cell concentration of more than 10⁵ cells per ml (preferably >5 10⁵ to 10⁶ cells per ml) is required for the effective cultivation of animal cells. If the cell concentration is lower the metabolites and allomones required for rapid growth are present in too low a concentration for the required high growth rates to be obtained.

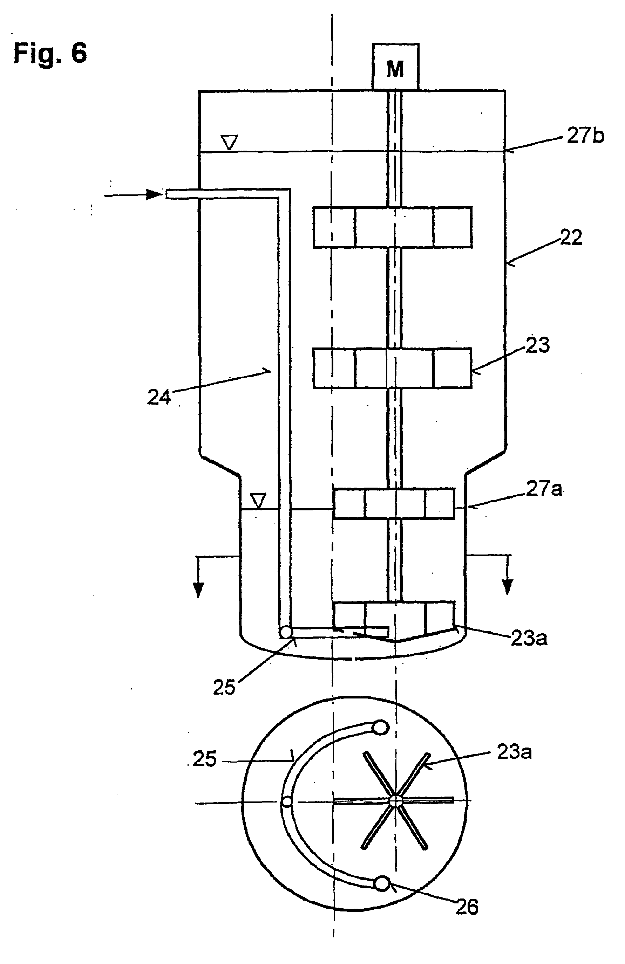

According to the invention the fermenter (Fig. 6) has a cross-section which is tapered in a downward direction. One special variant is cylindrical in shape both in the lower (27a) and the upper section (27b), the lower section having a smaller diameter. The lower section thus holds only approx. 1/6th of the total volume. A specially shaped transition fitting connects the lower to the upper section of the vessel.

Other reactors according to the invention have a conical shape which is tapered in a downward direction.

A multi-stage stirring system consisting of several large-area blade stirrers (23, 23a) and immersed baffles close to the stirrers is used for stirring. Aeration takes place via microspargers arranged close to the base of the vessel. According to the invention the stirrer system is designed in such a manner that the power introduced in each cross-section of the reactor is sufficient for the uniform distribution of the microbubbles and the movement of the cells. For this purpose, in accordance with the variable reactor cross-section, blade stirrers having a variable diameter and blade height and varying distances from each other are used.

As an alternative to the reactor system using baffles a reactor system without baffles and with an eccentrically arranged stirrer system (23) is used.

The culture in the pre-fermenter (9) can be operated successively as a batch, fed-batch and continuous perfusion process with or without cell retention. Continuous pre-culture operation is particularly advantageous since 3-4 fold higher cell densities are obtained than in batch fermentation processes and inoculation material for production fermenters can be provided over a longer period of time, so that the rapid start-up of any subsequent production process can take place.

In the continuous operation of the pre-culture process an analogous technical process is used to that of the production culture. The process set-up again consists of a number of apparatuses and a least a storage tank containing the nutrient medium, pumps and pipelines, the inoculation fermenter (9) which is then continuously perfused, throughflow heat exchangers and a cell retention system with or without a vibrator, all of the process components being designed in such a manner that low-shear cultivation with cell densities of higher than 10⁷ living cells per ml of reactor volume and a viability of higher than 90% can be achieved. 3. Sedimentation separator

The external sedimentation separators (4) developed for the process are characterized by a minimum volume for a given effective separator surface area, as a result of which the residence times of the cells outside the fermenter and the corresponding insufficient supply of oxygen can be reduced to a minimum.

The sedimentation separators used in the fermentation units according to the invention contain tubes or channels having a rectangular cross-section and arranged in parallel. The apparatuses according to the invention have preferably tube diameters or channel heights of 10 mm or less, a length of approx. 0.2 to 2.5 m or of approx. 0.2 to 1.5 m and an angle of inclination towards the horizontal of α = 40-65°.

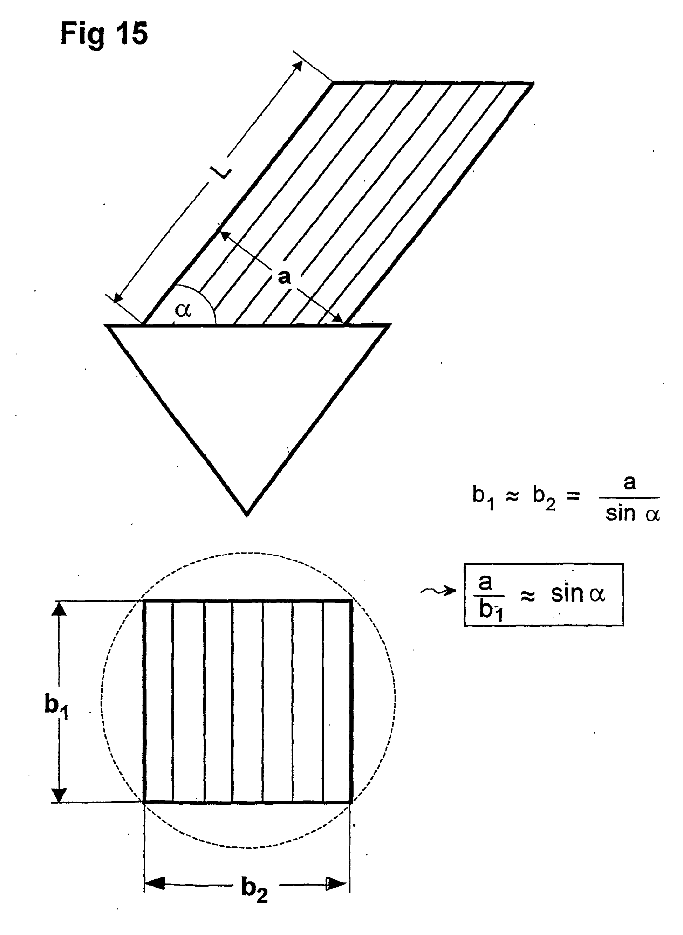

In the case of the separator with parallel channels having a rectangular cross-section the apparatus consists of a pressure-stable rectangular module (29) which is screwed to the concentrate return vessel (32, 44) arranged beneath it and to a covering plate via welded-on flanges. The rectangular module (29) contains grooves which determine the channel height and are arranged opposite each other on two sides at constant spacings and into which plates are inserted according to the invention which form the boundary to the individual channels. The cross-section of the module is preferably a rectangle whose height-to-width ratio (a/b₁) corresponds to the sine of the angle of inclination of the module (Fig. 15). This allows the surface area and the volume of the concentrate return vessel beneath the module to be as small as possible.

The module is stamped from a solid block of material or is welded together seamlessly from prefabricated U-shaped sections or from four plates.

The various structural elements are screwed together in such a manner that long-term sterility can be guaranteed with the aid of O-ring seals.

Sedimentation separators used in units according to the invention can be disassembled in such a manner that they can be readily cleaned and maintained. As far as the separator with parallel channels having a rectangular cross-section is concerned this also applies to the plates (30) forming the channels. They can be removed after the covering plate has been unscrewed and if necessary cleaned externally.

According to the invention, in large sedimentation separators the module is mounted in a rotatable fashion (4a) at the center of mass of the apparatus as a whole, so that the plates can be easily removed in a horizontal position and reinserted into the grooves of the module.

In order to reduce the residence time of the cells outside the fermenter the upward-facing surfaces of the sedimentation separator are fabricated with a high degree of surface smoothness, and preferably with a surface roughness of Ra < 0.25 µm, as defined by DIN 4768, which is equivalent to ISO 3274 and ASME B46.1-1995.. As an alternative hydrophobically coated surfaces_and surfaces with a lotus flower effect have proven suitable.

A lotus flower effect is understood to be the soil-repellant effect of certain surfaces due to a suitable combination of a well defined surface structure (roughness) and the properties of the material concerned, such as is observed for example in certain plants, such as for example in lotus flowers. A surface structure with microscopically small knobs made of a suitable material is for example considered to be responsible for the lotus flower effect. A suitable material employed is frequently a hydrophobic material.

### 3.1 Countercurrent sedimentation separator

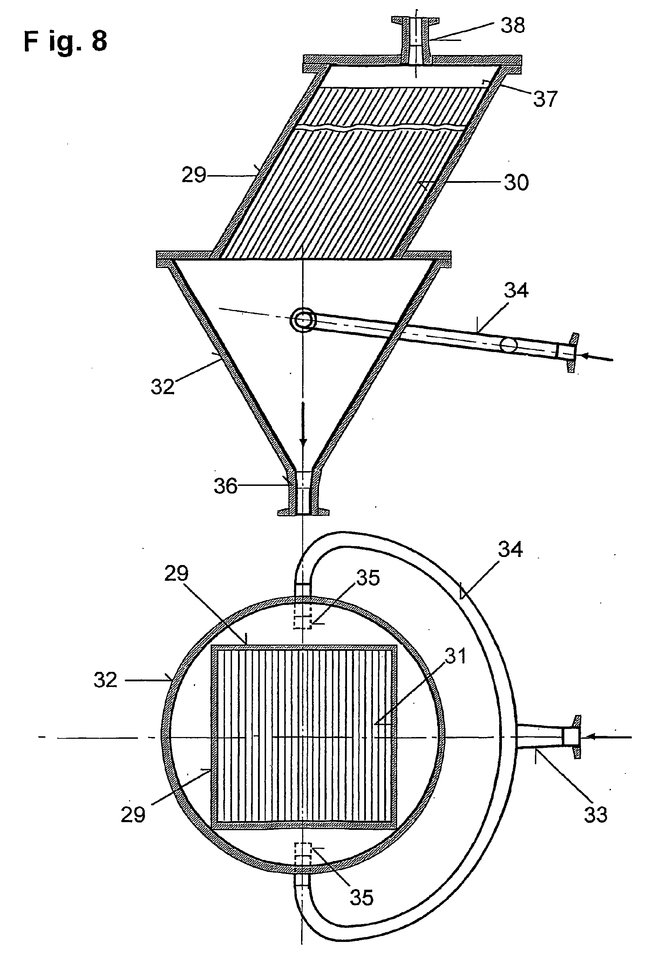

Countercurrent sedimentation separators (Fig. 8 to 12) consist of three parts: the module containing the channels (31), the concentrate return vessel (32) and the covering plate (29).

The suspension leaving the fermenter (2) is introduced beneath the inclined channels into a conical or pyramidal container (32) and the clear phase is removed above the channels (38).

The recycling outlet for the concentrate is incorporated centrally (36) in the receiving chamber at its lowest point so that the cell agglomerates sliding off the plates in a downward direction can be collected in the receiving chamber and recycled into the fermenter. Since in the receiving chamber (32) of the countercurrent sedimentation separator both the inflow of the cell suspension to be clarified and the return of the separated cell agglomerates formed in the channels take place and since according to the invention the volume of this vessel should be minimized, the flow characteristics of the vessel and the inflow pipelines have to be designed in a favorable manner. In particular when scaling up for the use of large apparatuses any additional promotion of fluctuations in speed (e.g. upon the onset of turbulence) by the inflowing stream must be avoided. Only in this way is it possible to ensure that the major portion of agglomerates separated in the receiving chamber is not resuspended and reintroduced into the channels (31). Unfavorably designed apparatuses displaying this phenomenon lead to a reduction in cell viability to below 80%.

Based on fundamental considerations with regard to fluid dynamics design principles which appeared to be appropriate for the receiving vessel were selected and optimized further by means of CFD (computational fluid dynamics) calculations and special experimental tests. The following design principles were accordingly developed according to the invention:
- the symmetrical arrangement of the inflow channels (34) for avoiding cyclic potential motion in the receiving chamber;
- the use of large inflow cross-sections to ensure that the inflow stream velocities are less than 0.1 m/s;
- the use of inflow diffusors with a small apex angle which produce a reduction in speed with low turbulence;
- the use of conical diffusors with half cone angles of less than 4° or alternatively of less than 6° and where flat diffusors are used a longitudinal differential of the cross-sectional area divided by the periphery (1/P dA/ds) of less than 0.1 (P: periphery [cm]; A: area [cm²]; s: length coordinate [cm]).
- the use of two radial, preferably tangential, inflow streams.

In the case of the radial inflow stream variant (Fig. 8) with two directly opposing inflow streams (35) which produce only one stagnation point flow in the center of the receiving chamber the entry points of the inflow streams are arranged at a clear distance from the channels and the concentrate recycling outlet. The distances are prefereably greater than 15 times the channel height or preferebly 10 times greater than the diameter of the concentrate recycling outlet. The inflow takers place at a geometrical height above the base of the receiving chamber which is greater the half and smaller the o.8 of the total height of receiving chamber.

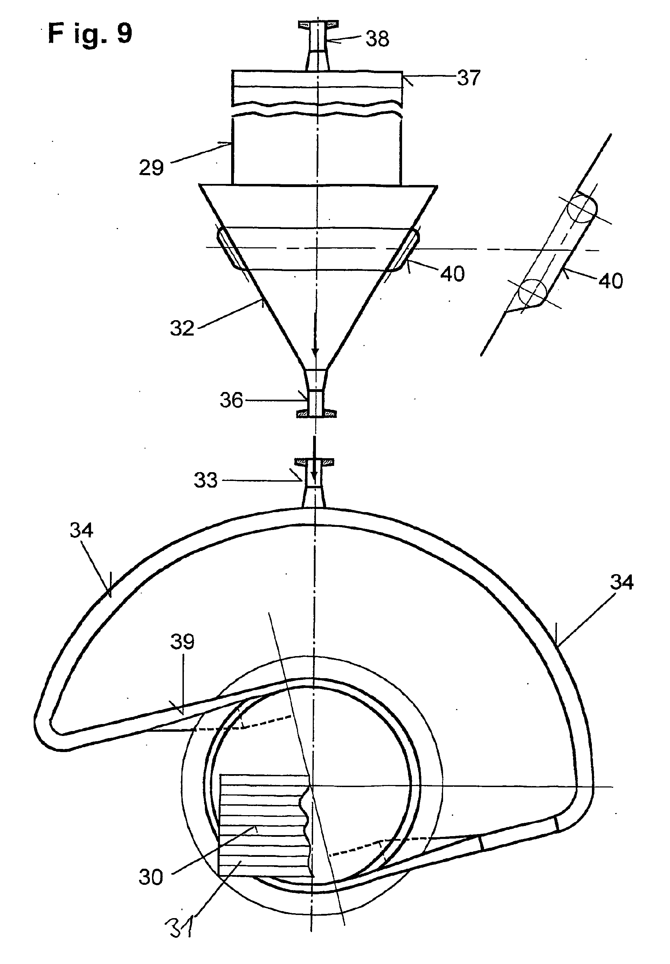

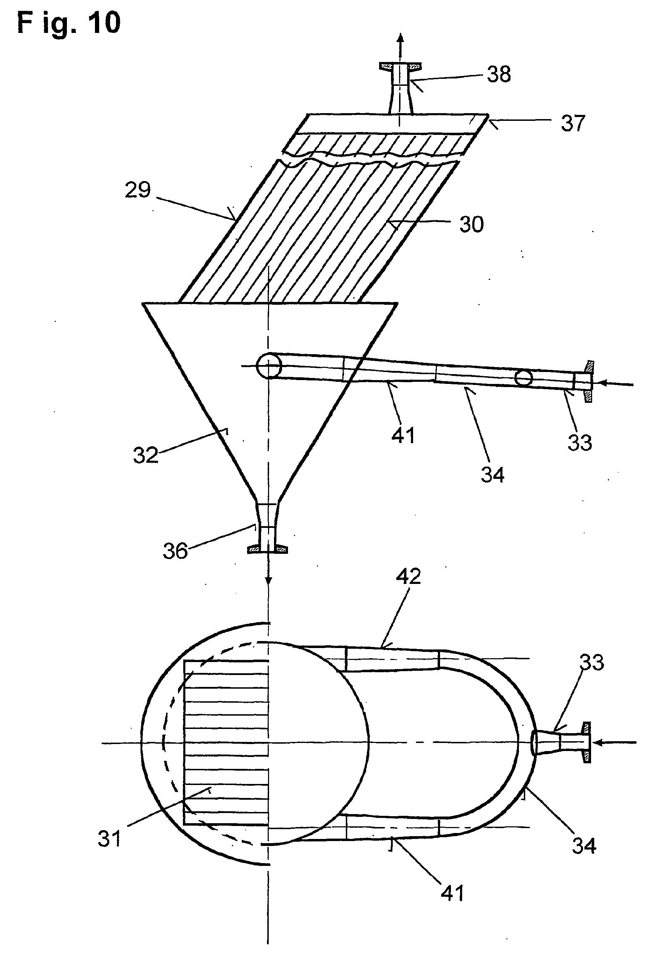

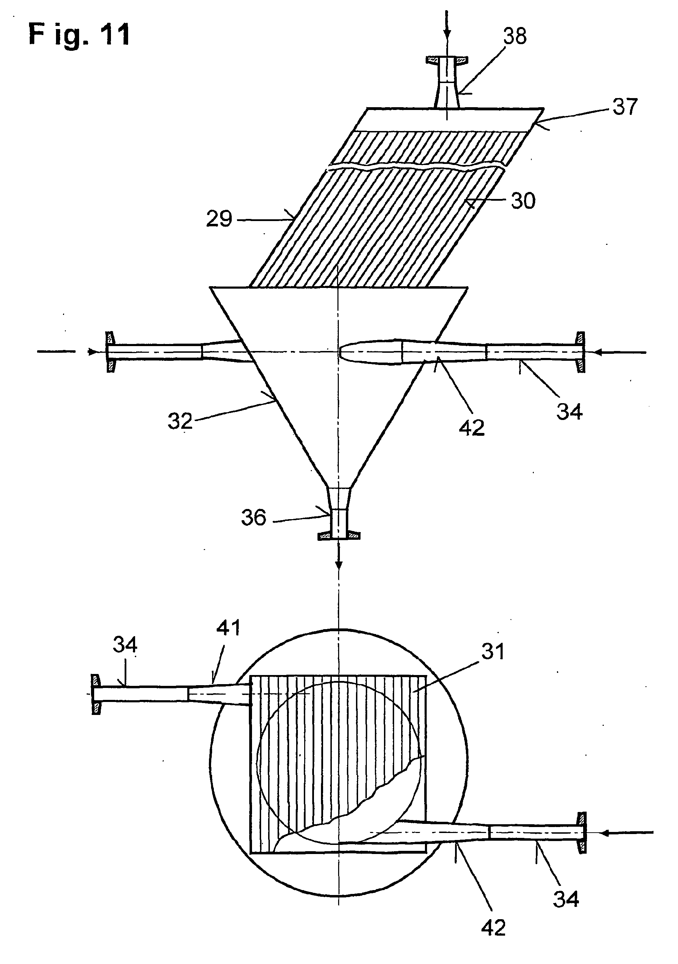

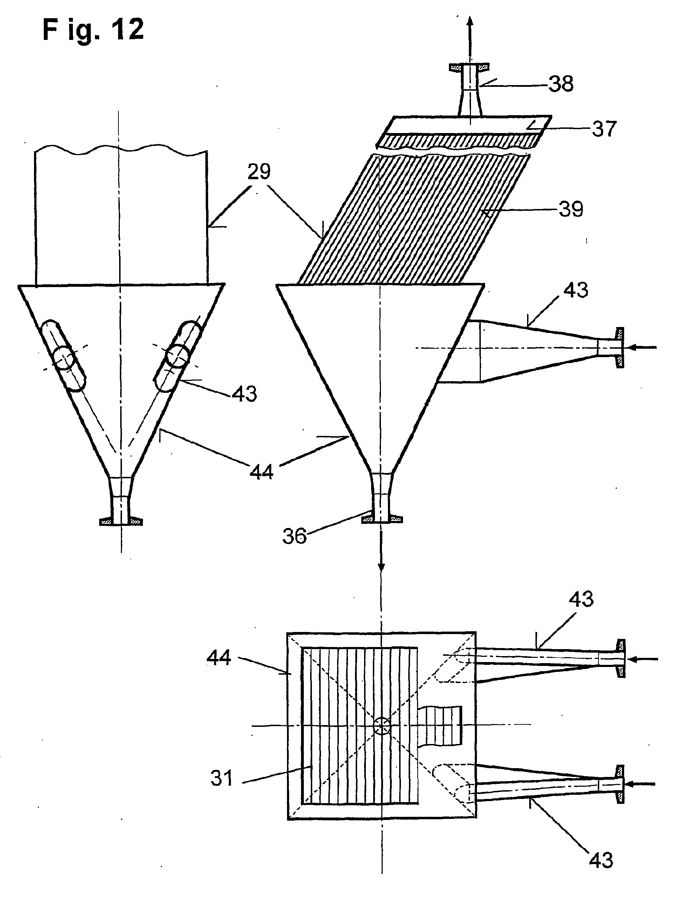

The inflow stream variants in which the inflow streams are arranged tangentially are distinguished by the arrangement of inflow streams in identical (Fig.9; Fig.11) or opposite directions (Fig.10; Fig. 12).

In the case of a tangential inflow stream and a conical receiving chamber the inflow can preferably take place in a ring channel (40 in Fig, 9) arranged outside the vessel.

### 3.2 Cross-flow sedimentation separator

In the case of cross-flow sedimentation separators the channels are designed according to the invention as rectangular channels. The apparatuses consist of a module comprising the channels, the covering plate and the concentrate return vessel. In the module the inflow stream from the fermenter is arranged to one side of the channels and the clear phase outlet on the opposite side. Inside the inflow and outflow chambers plates (49) can be built in for the improvement of the liquid distribution. The plates can be either flat plates or shaped. The plates are preferably arranged in close proximity and preferably perpendicular to the inflow and outflow channels. In order to ensure a uniform distribution of liquid over all the channels the inflow takes place via circular or flat inflow diffusors in a chamber upstream or downstream of the channels. According to the invention these flow regions are designed using special fluid dynamic tests in such a manner that non-detached, laminar flow is obtained at velocities of less than 0.1 m/s and uniform distribution takes place over the channels arranged in parallel.

In addition to the inflow of medium from the inflow channel it may be advantagous to supply a certain amount of fermentation broth directly to the conical or pyramidal receiving chamber (44). This procedure reduces the retention time of cells in the conical or pyramidal receiving chamber (44).

Beneath the inclined channels a conical or pyramidal vessel is located in which the concentrate recycling outlet is incorporated centrally at the lowest point.

The cross-flow separators have the following advantageous properties:
- The separation and recycling of the cells is not hindered, as in the case of countercurrent systems, by countercurrent flow.
- The codirectional flow of the descending concentrate promotes the downward slide of the cells on the plates. This is particularly the case where the ratio of the concentrate return stream to the clear phase stream q_{R}/q is larger than 2.
- Given an identical channel cross-section the concentrate collection vessel can be smaller than in the case of the countercurrent separator, since according to the cross-flow separator design inflow does not take place in the concentrate collection vessel.
- The receiving chamber upstream of the separating channels and the outflow chamber downstream thereof can have such dimensions that their volumes when scaling up the process do not increase overproportionally as the separator area increases, which, in comparison with the countercurrent separator represents a crucial advantage of the cross-flow separator.
- In this separator type the channel length can be smaller than in the countercurrent sedimentation separator. The smaller channel length, the non-existence of countercurrent flow and the return stream promoting the downward slide of the cells reduce the residence time of the cells in the separator.

The cross-flow separator is therefore preferably used for extremely high cell densities.

### 4. Vibration

In order to accelerate the recycling of the cells and to prevent fouling of the channels pneumatically or electrically operated vibrators can be used. They are preferably fixed at the top or on the flange of the concentrate return vessel.

The vibration intensity, amplitude and frequency is adapted to the operating and culture conditions and the cell retention system concerned. The vibration intensity is preferably 0.1 to 0.3 g, the amplitude 0.1 to 1 mm and the frequency 20 to 50 Hz.

### 5. Use of a cyclone, an ultrasonic separation system and a second sedimentation apparatus

For the preliminary separation and reduction of the cell mass to be separated in the sedimentation separator it can be recommendable to arrange a cyclone or an ultrasonic separation system upstream of the sedimentation separator. Such a cyclone can separate off up to 50% of the cell mass and more at a lower inflow speed, i.e. a lower degree of stress on the cells. A similar effect can be obtained with the ultrasonic separation systems available on the market (e.g. Biosep from Applikon, Schiedam, the Netherlands), which allow partial separation at technically advantageous rates of perfusion.

Due to the lower cell concentration, improved cell recycling and higher perfusion rates are obtained in the subsequent cell separation process in the sedimentation separator. It is thus possible to operate the fermenter at higher cell densities and to increase the productivity of the process.

The separation of large cell agglomerates by a smaller sedimentation apparatus upstream or preferably downstream of the sedimentation separator has a similarly positive effect. Since the large agglomerates enclose a percentage-wise larger number of dead cells due to the insufficient supply of substrate (limited diffusion) these dead cells are removed to a larger extent than living cells, the total cell count is reduced and the vitality increased.

### 6. Pumps, pipelines and heat exchangers

Three pumps are required for continuous operation of the fermentation process. The first pump is responsible for the introduction of the substrate into the fermenter, the second pump transports the cell suspension from the fermenter to the sedimentation separator and the third pump from the separator to the harvest vessel. Low-shear pumps have to be used for the two pumps transporting the cell suspension and in particular for pump 2, so that no reduction in cell vitality takes place. According to the invention low-speed positive-displacement pumps which transport the suspension with a low degree of pulsation are used. Hose pumps with large pump tubes and other seal-free positive-displacement pumps which cause particle stress of less than 0.01 N/m² or less than 0.004 N/m² have proven suitable for sterile long-term operation.

Spiral heat exchangers which allow effective heating at low residence times are used for cooling the cell suspension to temperatures lower than the fermenter temperature before it enters the separator and for heating it to the fermenter temperature during recycling to the fermenter.

The pipelines and connection fittings to the apparatuses are designed in such a manner that only low flow velocities above the deposition limit of the cells prevail in the pipelines, bends in the pipes are reduced to a minimum and only gradual cross-sectional widening takes place. Elbow fittings having ratios of curvature radius to pipe diameter of >2 are preferably used. The cross-sectional widening comprises diffusors with semi-cone angles of up to 6°, preferably up to 4°. The reduction in cross-section and the corresponding acceleration of flow must be limited so that the forces of inertia occurring as a result of the acceleration of flow remain small.

The invention relates to a unit for carrying out continuous high cell density fermentation containing a pre-culture fermenter (9), a substrate storage tank (1), a production fermenter (2), a sedimentation separator (4) and a harvest vessel (6) in which the sedimentation separator has a separator area based on the separator volume of Aₜₕ/V_{S}>30 m²/m³ or more preferred of Aₜₕ/V_{S}>70 m²/m³. Even more preferred are separator surface areas based on the separator volume of Aₜₕ/V_{S} = 50 to 100 m²/m³. Preferably the sedimentation separator is a counter-current sedimentation separator. In a preferred embodiment the separator has an absolute surface area of Aₜₕ = 0.5 m² to 10 m² and still has the high Aₜₕ/V_{S} as described above.

The invention also relates to a unit for carrying out continuous high cell density fermentation containing a pre-culture fermenter (9), a substrate storage tank (1), a production fermenter (2), a sedimentation separator (4) and a harvest vessel (6) in which the sedimentation separator has a conical (32) or pyramidal receiving chamber (43) and the inflow into the receiving chamber of the sedimentation separator takes place via at least two conduits arranged radially (35), tangentially in an identical direction (39, 42) or tangentially in opposite directions (41, 43), the conduits being distributed in a regular fashion over the cross-section.

The invention also relates to the abovementioned unit in which the inflow into the sedimentation separator takes place via the conduits arranged tangentially in an identical direction in a ring channel (40) arranged outside the receiving chamber.

In addition the invention relates to units for high cell density fermentation in which the inflow into the sedimentation separator takes place via circular diffusors (35) having a semi-cone angle of at most 6°, more preferred 4°, or via flat diffusors (39) having a longitudinal differential of the periphery-related cross-sectional area of 0.1 or less, at velocities of at most 0.1 m/s.

In addition the invention relates to a unit for carrying out continuous high cell density fermentation containing at least one sedimentation apparatus in which the inlet for the inflow from the fermenter (45, 48) and the outlet for the clear phase (46) are arranged on either side of the sedimentation channels and the concentrate collection chamber (44) consists of a pyramidal or conical container arranged symmetrically beneath the sedimentation channels and in which cross flow is generated in the channels vertically to the biomass sliding down the channels.

In addition the invention relates to the above units in which the ratio of the width (b₁) of the module and the length (L) of channels of the cross current sedimentation separator is close to 1.

In addition the invention relates to the above units in which the inflow (35) into the receiving chamber of the sedimentation separator takes place at a geometric height above the base of the receiving chamber which is more than half the total height of the receiving chamber and less than 0.8 of the total height of the receiving chamber.

One variant of the invention contains a sedimentation separator consisting of a rectangular module (29) in which individual channels (31) are spatially separated from each other by plates (30) and said plates are guided and held in grooves in the module and they can if necessary be assembled or disassembled.

The invention also relates to the above units in which the rectangular sedimentation channels or sedimentation tubes of the sedimentation separator are 50 cm in length or longer and the corresponding channel heights are smaller than or equal to 10 mm.

The most preferred channel heights are those between 4 and 6 mm. It has been found that channel heights between 4 and 6 mm represent the optimum heights with respect to settling characteristics and settler volume. Smaller channel heights lead to more unfavourable settling characteristics while large channel heights lead to an unfavourable high volume of the settler. These very small channel heights are different from those known from state-of-the-art settlers, e.g., such settlers used for sludge recycling in wastewater treatment plants.

The invention also relates to the above units in which the rectangular sedimentation channels or sedimentation tubes of the sedimentation separator are 50 cm in length for the counter current separator and 20 cm in length for the cross current separator or longer and the corresponding channel heights are smaller than or equal to 10 mm.

In addition the invention relates to the above unit in which the parallel plates or tubes of the sedimentation separator are arranged inside a rectangular module whose cross-sectional height (a) to cross-sectional width (b₁) ratio corresponds approximately to the sine of the angle between the horizontal and the angle of inclination of the module (Figure 15 ) in its assembled state.

In addition the invention relates to a unit as described above in which the parallel plates or tubes of the sedimentation separator have a surface roughness of less than Ra = 0.25 µm on their upward-facing surfaces or these surfaces are hydrophobically coated or they have a surface finish with a lotus flower effect.

The invention also relates to a unit in which the parallel plates or tubes of the sedimentation separator can be subjected to vibrations of a specific frequency and amplitude.

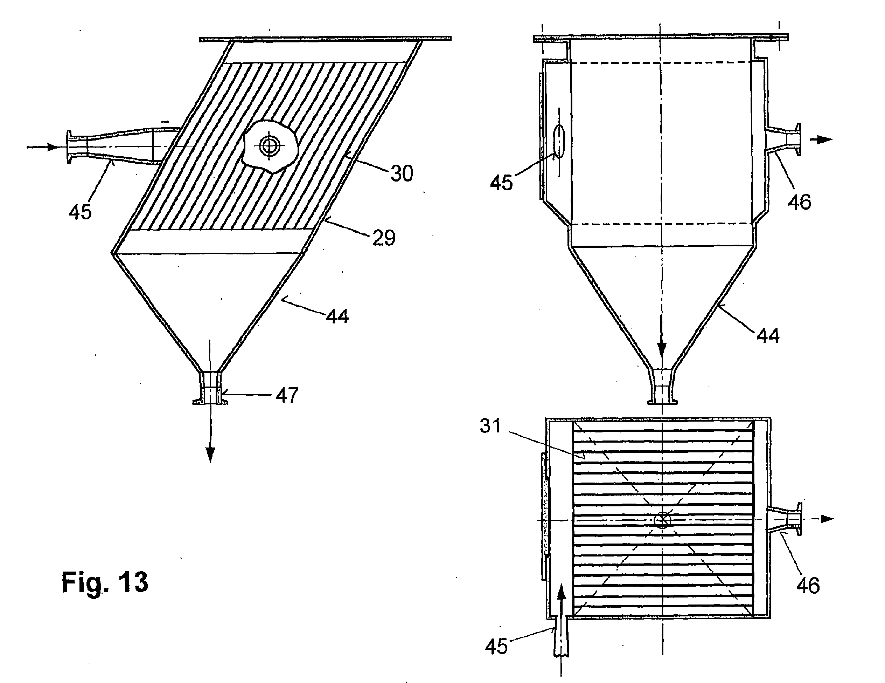

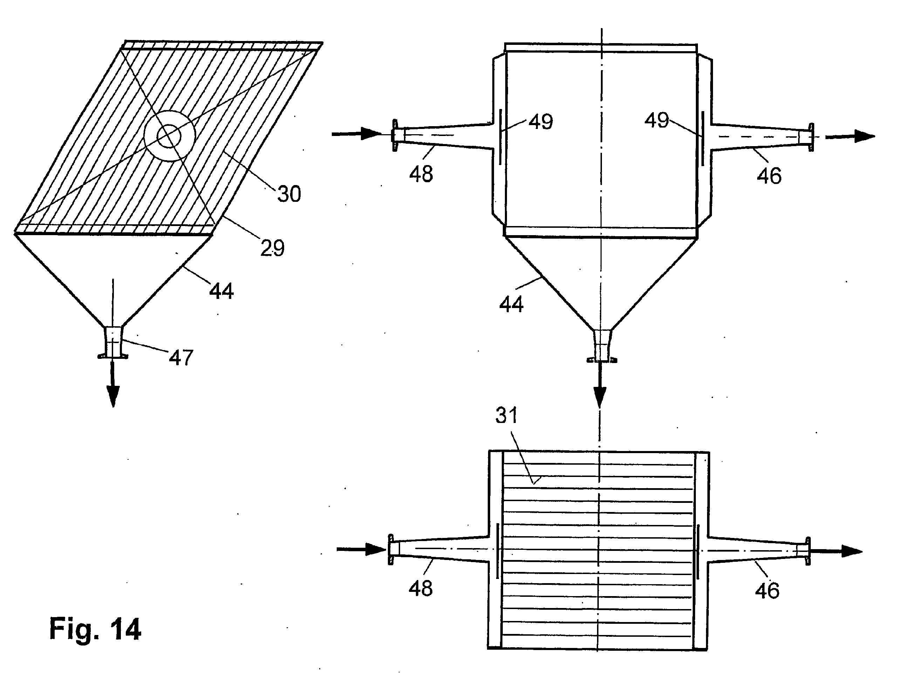

In addition the invention relates to a unit for carrying out continuous high cell density fermentation containing a pre-culture fermenter (9), a substrate storage tank (1), a production fermenter (2), a sedimentation separator (4) and a harvest vessel (6) in which the sedimentation separator is operated according to the cross-flow principle (Fig. 13, 14).

The invention also relates to units for carrying out continuous high cell density fermentation processes in which the pre-culture fermenter (9) has a cross-section which is tapered in a downward direction, the stirrer of the pre-culture fermenter (23) is suspended in an eccentric fashion and the aeration of the pre-culture is carried out by a microsparging aeration unit (25).

The invention also relates to units for carrying out continuous high cell density fermentation processes in which a large-area anchor stirrer (13) and an axially wound bubble-free aeration system (14) are used in the production fermenter. An aeration unit (17) can be additionally incorporated in these units and the stirrer blades of the anchor stirrer can be tapered in the region close to the base (13a).

Instead of the anchor stirrer, also gate stirrers can be used. Anchor or gate stirrers have a preferred diameter of larger than half the vessel diameter, more preferred 70% of the vessel diameter or 80% of the vessel diameter.

The invention also relates to units for carrying out continuous high cell density fermentation processes in which a large-area blade stirrer (18) and baffles (19) which are inclined in a peripheral direction and are arranged at a distance from the wall, the fermenter base and the liquid surface and an aeration ring for microsparging (17) are arranged in the production fermenter.

The invention finally also relates to units for carrying out continuous high cell density fermentation processes in which a large-area blade stirrer (21) is arranged in an eccentric fashion in the production fermenter as well as to units in which an aeration ring for microsparging (17) is additionally incorporated in the production fermenter.

The invention also relates to units in which a hydrocyclone (11) or an ultrasonic separation system is arranged upstream of the sedimentation separator and to units in which an agglomerate separator (12) is arranged downstream of the recycling outlet of the sedimentation separator (36).

An agglomerate separator in the context of the invention is a device for removing cell agglomerates from a liquid medium which operates for example according to the sedimentation principle. An agglomerate separator allows the separation of cell agglomerates from a continuously flowing media stream.

The invention also relates to units in which a throughflow heat exchanger (3) is arranged in each case between the outlet of the fermentation tank and the inlet of the sedimentation separator and between the concentrate outlet of the sedimentation separator (36) and the recycling inlet of the fermentation tank.

The invention does however also relate to a process for carrying out continuous high cell density fermentation processes in which a unit according to the invention is used. This process can be carried out using a single-stage pre-culture in which the pre-culture is carried out at least intermittently as a fed-batch process and in which the pre-culture volume increases to at least 5 times the initial volume due to the fed batch. The pre-culture can also be carried out intermittently as a continuous process with cell recycling.

In the process according to the invention it is also possible for a pre-culture to be carried out as a continuous process with cell recycling at the same time as the production culture in order to provide seed over a relatively long period of time for the next main culture or main cultures carried out in parallel.

### (Brief description of the figures)

In the following, working examples of the invention are described in more detail with the aid of schematic drawings.
Fig. 1: The process set-up with a substrate storage tank (1), pumps (8), pipelines (7), the production fermenter (2), throughflow heat exchangers (3), a cell retention system (4) with a vibrator (5) and a harvest vessel (6), a pre-culture fermenter (9) and a stock culture tube (10).
Fig. 2: Part of the process set-up comprising pumps, throughflow heat exchangers, a cyclone or ultrasonic separation system and a cell retention system mounted at the center of mass and having a vibrator and a sedimentation tank arranged downstream.
Fig. 3: A stirred fermenter of type A comprising an anchor stirrer and an aeration cage for silicone tube aeration.
Fig. 4: A stirred fermenter of type B comprising large-area blade stirrers, immersed, inclined baffles close to the stirrers and microspargers arranged close to the base.
Fig. 5: A stirred fermenter of type C comprising large-area blade stirrers which are arranged in an eccentric fashion and microspargers arranged close to the base.
Fig. 6: An inoculation fermenter.

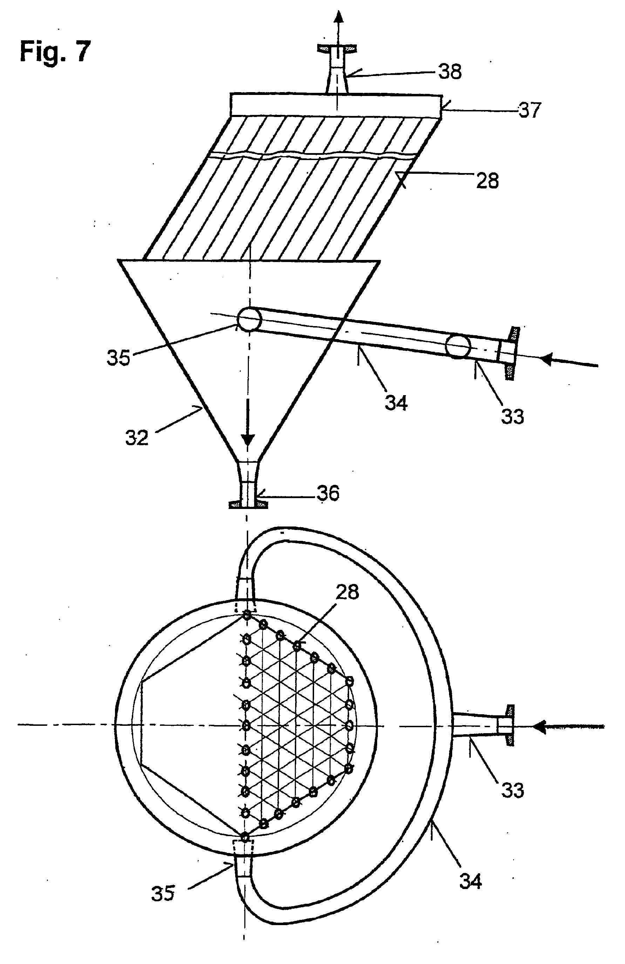

Fig. 7: A countercurrent sedimentation separator with tubes arranged in parallel and radial inflow into the conical receiving chamber.
Fig. 8: A countercurrent sedimentation separator with channels arranged in parallel and radial inflow into the conical receiving chamber.
Fig. 9: A countercurrent sedimentation separator with channels arranged in parallel and tangential inflow in an identical direction in a ring channel (40) arranged on the outer side of the conical receiving chamber.
Fig. 10: A countercurrent sedimentation separator with channels arranged in parallel and tangential inflow in opposite directions into a conical receiving chamber.
Fig. 11: A countercurrent sedimentation separator with channels arranged in parallel and two separate oppositely directed tangential inflow streams into a conical receiving chamber.
Fig. 12: A countercurrent sedimentation separator with channels arranged in parallel and two separate oppositely directed tangential inflow streams into a pyramidal receiving chamber.
Fig. 13: A cross-flow sedimentation separator with channels arranged in parallel and an inlet (45) for the inflow from the fermenter and a clear phase outlet (46) arranged on either side of the channels and a pyramidal receiving chamber (44) beneath the channels.
Fig. 14: A cross-flow sedimentation separator with channels arranged in parallel and an inlet (48) for the inflow from the fermenter and a clear phase outlet (46) arranged on either side of the channels and a conical or pyramidal receiving chamber (44) beneath the channels. Plates (49) arranged in close proximity of the inflow channel (48) and the outflow channel (46) which are preferably mounted perpendicular to the inflow and outflow channels.
Fig. 15: Geometry of the sedimentation separator.

### Examples

### Example 1

### Continuous cultivation of hybridoma cells with membrane based oxygen supply and additional sparging and counter-current cell retention using a 40 liter fermenter

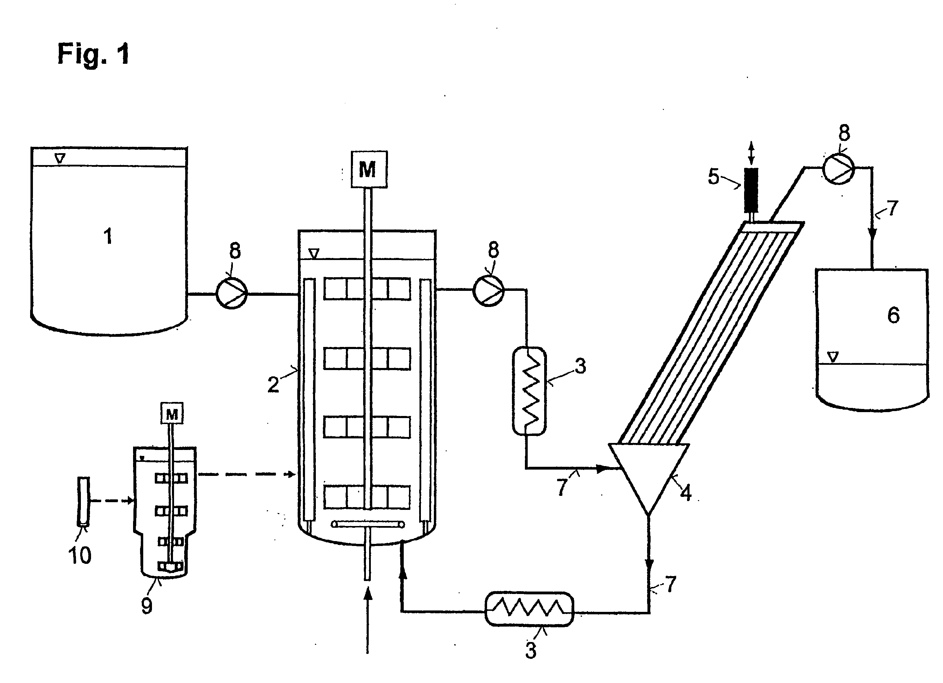

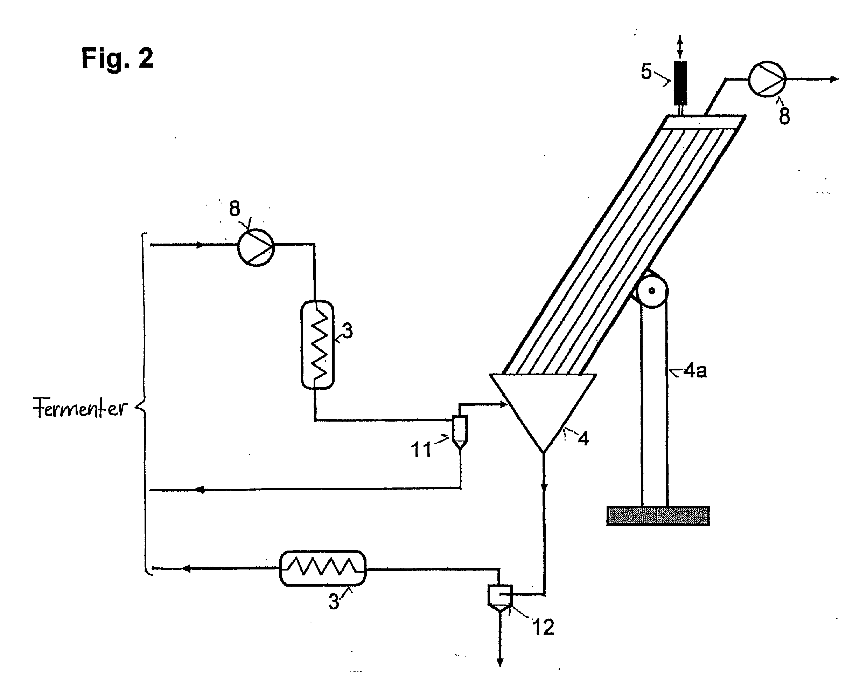

The process set-up according to Figs. 1 and 2 consists of a storage tank (1) containing the nutrient medium with 1g/L human serum albumin (HSA), three pumps with pump rates of 3-30 l/h, of which at least those coming into contact with the cell suspension are low-shear tube pumps and pipelines (7), the continuously perfused fermenter (2) having a working volume of 40 liters, a gravity countercurrent sedimentation separator (4) according to Fig. 8 with a theoretical separating surface area of Aₜₕ = 0.56 m² (Aₜₕ= z b₁ L cos α ; z: number of channels, b₁: channel width, L: channel length, α: inclination of channels to the vertical) at at a channel length of L=630mm and the further dimenions z=16; b₁=111mm; α=60° and a pneumatically operated vibrator (5) which subjects the separator to vibration with accelerations of b = 0.1 g.

The counter-current separator is high efficient due to the high specific settling area of Aₜₕ/Vₛ 63 m²/m³ based on the separator volume.

The fermenter is a stirred fermenter according to Fig. 3, having a low-speed anchor stirrer (13) and a silicone tube stator (14) having a specific surface area of 65 m²/m³. The four blades of the anchor stirrer (13) are at a distance of 8 mm from the silicone tubes (16) and extend over the entire height of the stator (14). Additional to the silicon membrane a ring sparger (17) according to Fig. 3 with holes of 0.5 mm is arranged close to the bottom. If the cell concentration exceeds 2 ·10⁷ cells/ml additional oxygen sparging up to superficial gas velocities up to 2 m/h is applied.

After the start-up phase of approx. 12 days cell concentrations of 30 to 50 ·10⁷ viable cells/mL can be reached. This quasi steady state condition of the cell-fermenter-settler system is obtained at perfusion rates of 6,9 fermenter volumes per day. The cell separator used, at this perfusion rate, has an average degree of retention for living cells of R = 94,5 % and an average degree of retention for dead cells of R_{d} = 92 % and is just capable of guaranteeing such a cell concentration in long-term operation over a long period with cell viabilities (ratio viable cell number to total cell number) between 75 and 85% and antibody concentrations of 100mg/L. After 70 days the cultivation was stopped.

### Example 2

### Continuous cultivation of BHK-cells with membrane based oxygen supply and a counter-current cell retention system using a 100 l fermenter.

The process set-up according to Figs. 1 and 2 consists of a storage tank (1) containing the nutrient medium with 1 g/l HSA, three pumps with pump rates of 20 to 100 l/h, of which at least those coming into contact with the cell suspension are low-shear tube pumps and pipelines (7) with diameters of larger than 10 mm, the continuously perfused fermenter (2) having a filling volume of 100 l, throughflow heat exchangers (3) with specific heat exchange surface areas of larger than 300 m²/m³, a gravity counter-current sedimentation separator (4) with a theoretical separating surface area of Aₜₕ = 1,4 m² at a channel length of L=960 mm and the further dimenions z=20; b₁=148mm; α=60° (cf. Fig. 8) and a pneumatically operated vibrator (5) which subjects the separator to vibration with accelerations of b = 0.2 g The counter-current separator is highly efficient due to the high specific settling area of Aₜₕ/V_{S}=77 m²/m³. The fermentation broth with a temperature of 37°C coming from the fermenter is cooled for entering the cell-separator down by the heat exchanger to 20°C and heated up again to 37°C before entering the fermenter.

The fermenter is a stirred fermenter according to Fig. 3 which is stirred by a low-speed anchor stirrer (13) and a silicone tube stator (14) having a specific mass transfer surface area of 33 m²/m³. The four blades of the anchor stirrer (13) are at a distance of 10 mm from the silicone tubes (16) and extend over the entire height of the stator (14). At a stirrer rotation speed of 20 r.p.m., which corresponds to a power input of 15 W/m³ an oxygen input which is sufficient for the cultivation of 1.5 to 2 x 10⁷ living BHK cells/ml is obtained when using pure oxygen and an inner tube pressure of 2 bars.

For the supply of nutrients for 1.5 to 2 x 10⁷ living BHK cells/ml a perfusion rate of 10 fermenter volumes/d is necessary. The cell separator used has an average degree of retention for living cells of R = 97% and at an observed growth rate of µ = 0.4/d is just capable of guaranteeing such a cell concentration in long-term operation over a period of 3 months with a cell viability larger than Vi = 90%.

This high cell viability of culture could obtained by the lower temperature inside the cell separator.The steady state cultivation conditions are obtained after approx. 10 days.

### Example 3

### Continuous cultivation of CHO-cells with mico-sparging and a counter current cell retention 200L fermenter

The process set-up is according to Fig.1 and 2 and consists of a storage tank (1) containing the nutrient with medium 1 g/l pluronic, three pumps (8) with transporting capacities of 20 to 100 l/h, of which at least those coming into contact with the cell suspension are low-shear displacement pumps (8) and pipelines (7) with diameters of larger than 15 mm, the continuously perfused fermenter (2) having a filling volume of 200 l, throughflow heat exchanger (3) with specific heat exchange surface areas of larger than 300 m²/m³, a gravity counter-current sedimentation separator according to Fig 14 with a theoretical separating surface area of Aₜₕ=2.4 m² at a channel length of 960 mm and the further dimenions z=26; b₁=194mm; α=60° according to Fig.11 and a electric driven vibrator which subjects the separator to vibration with acceleration of 0.2 g.

The counter-current separator is high efficient due to the high specific settling area of Aₜₕ/Vₛ= 84 m²/m³ based on the separator volume.

The fermenter is a stirred fermenter according to Fig. 4 which is stirred by a large area, multi step bladed impeller (18) with a diameter ratio 0.6, inclined baffles (19) which have no contact with the wall, the liquid surface and the bottom. A ring sparger equipped with eight metal sinter plates, 0.5 µm pore size and 10 mm in diameter, is arranged close to the bottom. At a superficial oxygen velocity of only 0.2 m/h and a stirrer rotational speed of 25 r.p.m., which corresponds to a power input of 9 W/m³, an oxygen input which is sufficient for the cultivation of 4 x 10⁷ living BHK cells/ml is obtained.

For the supply of nutrients for 4 x 10⁷ living CHO cells/ml a perfusion rate of 4 fermenter volumes/d is necessary. For this perfusion rate has the cell separator used a average degree of retention for living cells of R = 90%. With the observed growth rate of the cells of µ = 0.4/d it is possible to guarantee such a cell concentration in long-term operation over a period of 110 days. The viability of cells remains larger than 90% over the whole cultivation time. The steady-state conditions with high cell densities are obtained after a start phase of 15 days.

Example 4:

### Continuous cultivation of BHK-cells in HSA free media with mico-sparging and a cross- current cell retention: 200L fermenter

The process set-up is according to Figs.1 and 2 and consists of a storage tank (1) containing the nutrient with medium 1g/l pluronic, three pumps (8) with transporting capacities of 75 to 300 l/h, of which at least those coming into contact with the cell suspension are low-shear displacement pumps (8) and pipelines (7) with diameters of larger than 15 mm, the continuously perfused fermenter (2) having a filling volume of 200 l, throughflow heat exchanger (3) with specific heat exchange surface areas of larger than 200 m²/m³, a gravity cross-current sedimentation separator according to Fig 14 with a theoretical separating surface area of Aₜₕ=2,8 m² at a channel length of 345 mm and the further dimenions z=46; b₁=345mm; α=60° and a electric driven vibrator which subjects the separator to vibration with acceleration of 0.1g.

The cross-current separator according to Fig.14 is high efficient due to the high specific settling area of Aₜₕ/Vₛ=75 m²/m³ based on the separator volume and the much shorter channel length as the counter-current system used in example 1-3.

The fermenter is a stirred fermenter according to Fig. 3 which is stirred by a large area multi step bladed impeller (18) with a diameter ratio 0.6, inclined baffles (19) which have no contact with the wall, the liquid surface and the bottom. A ring sparger equipped with eight metal sinter plates, 0.5 µm pore size and 10 mm in diameter, is arranged close to the bottom. At a superficial oxygen velocity of only 0.15 m/h and a stirrer rotation speed of 25 r.p.m., which corresponds to a power input of 9 W/m³ an oxygen input which is sufficient for the cultivation of 3 x 10⁷ living BHK cells/ml is obtained.

For the supply of nutrients for 3 x 10⁷ living BHK cells/ml a perfusion rate of 15 fermenter volumes/d is necessary. The cell separator used has a average degree of retention for living cells of R = 96,8 % and at the observed growth rate of the cells of µ = 0.5/d is just capable of guaranteeing such a cell concentration in long-term operation over a period of 100 days. The viability of cells remains larger than Vi>95% over the entire cultivation time. The steady state cultivation conditions are obtained after a start phase of 12 days.

The higher perfusion rate, the higher growth rate and the increased viability of the culture as compared to examples 1-3 is caused by the cross-current separator principle which leads to shorter residence time of cells inside the separator.

The process includes an inoculation fermenter specially designed and operated according to Fig. 6, whose filling volume is less than 6% of the production fermenter volume and which allows an approx. 120 or 150-fold increase in the cell count in two weeks. The innoculation itself is started by innoculation with a 50 ml vial and a starting volume of 2L. After 2 days the volume is filled up to 5L and after 4 days up to 12L. In the first 4 days the fermenter is running in a batch mode and after 5 days in a continuous mode The final cell concentration of 2 to 2.5·10⁷ viable cells/mL, sufficient for innoculation of the 200L fermenter, is obtained in only 12 days. The cell retention system used for the continuous fermentation of innoculation fermenter is designed according to Fig. 9 with a theoretical separating surface area of Aₜₕ=0,12 m² at a channel length of 500 mm.

### Example 5: Determination of the turbulent shear stress τ.

Low-shear pumps are used in the process. The pumps are selected by means of a special pump test, in which the stress caused by the pump is tested in a model experiment using a suitable model particle system of a known interfacial surface tension σ in relation to the aqueous fermentation medium. The equilibrium particle diameter d_{P} which establishes itself after long periods of pumping gives the maximum turbulent shear stress τ = σ /d_{P} exerted by the pump. If the equilibrium diameter of the particle system differs from that of the cells or cell agglomerates used during fermentation, the stress caused by the pump can be determined by applying the laws of the theory of isotropic turbulence. The turbulent shear stress is calculated from the laws of the dissipation range, according to which τ_{cell}/ τ_{model particles} = d_{cells}/d_{model particles}.

## Claims

1. A unit for carrying out continuous high cell density fermentation containing a pre-culture fermenter (9), a substrate storage tank (1), a production fermenter (2), a sedimentation separator (4) and a harvest vessel (6), **characterized in that** the sedimentation separator has a separator surface area of Aₜₕ/Vₛ≥30 m²/m³ based on the separator volume and the sedimentation separator has a conical or pyramidal receiving chamber (32, 44) and the inflow into the receiving chamber of the sedimentation separator takes place via at least two conduits arranged radially (35), tangentially in an identical direction (39, 34) or tangentially in opposite directions (41, 42), the conduits being arranged in a regular fashion over the cross-section..

2. A unit for carrying out continuous high cell density fermentation according to claim 1, **characterized in that** the sedimentation separator has a separator surface area of Aₜₕ≥ 0.5 m² and at the same time either a specifc surface area of Aₜₕ/Vₛ ≥ 30 m²/m³ (based on the separator volume) or a specific surface area of Aₜₕ/V ≥ 5 m²/m³ (based on the fermenter volume) while the unit can be run at perfusion rates in the range of 5 to 15 fermenter volumes per day.

3. A unit for carrying out continuous high cell density fermentation according to claim 1 and 2, **characterized in that** the sedimentation separator has a separator surface area of Aₜₕ/Vₛ 50 - 100 m²/m³ based on the separator volume

4. A unit according to any of Claims 1 to 3, wherein the inflow into the sedimentation separator (4) takes place via the conduits arranged tangentially in an identical direction in an annular channel (40) located outside the receiving chamber.

5. A unit according to at least one of Claims 1 to 4 in which the inflow (35) into the receiving chamber of the sedimentation separator (4) takes place at a geometrical height above the base of the receiving chamber which is greater than half the total height of the receiving chamber and smaller than 0.8 times, more preferred smaller than 0.75 times, the total height of the receiving chamber.

6. A unit according to at least one of Claims 1 to 4 in which the sedimentation separator is operated according to the cross-flow principle.

7. A unit according to claim 6 in which an additional inflow channel transports fermentation medium directly into the receiving chamber (33, 44) thereby reducing the retention time of cells in said receiving chamber.

8. A unit according to at least one of claims 1 to 7 in which the parallel plates or tubes of the sedimentation separator are arranged inside a rectangular module whose cross-sectional height to cross-sectional width ratio corresponds approximately to the sine of the angle α between the horizontal and the angle of inclination of the module in its assembled state.

9. A unit according to at least one of claims 1 to 8 in which the Inflow into the sedimentation separator takes place via circular diffusors (35) having a half cone angle of at most 6° or via flat diffusors (39) having a longitudinal differential of the cross-sectional area divided by the periphery (1/P dA/ds) of 0.1 or less, at velocities of at most 0.1 m/s.

10. A unit according to at least one of claims 1 to 9, wherein said sedimentation separator consists of a rectangular module (29) in which individual channels (31) are spatially separated from each other by plates (30) and said plates are guided and held in grooves in the module and said plates can if necessary be assembled or disassembled.

11. A unit according to at least one of claim 1 to 5 and 8 to 10 in which the inclined rectangular channels or tubes of the sedimentation separator are 50 cm in length or longer and the corresponding channel heights are smaller than or equal to 10 mm.

12. A unit according to at least one of claims 1 to 5 and 8 to 10 in which the inclined rectangular channels or tubes of the sedimentation separator are 50 cm in length or longer and the channel heights are 4 to 6 mm.

13. A unit according to at least one of claim 6 to 7 in which the cross-current sedimentation separator has inclined rectangular channels which are 20 cm in length or longer, and the channels are 10 mm or less in height

14. A unit according to at least one of claim 6 to 7 in which the cross-current sedimentation separator has inclined rectangular channels which are 20 cm in length or longer, and the channels heights are 4 to 6 mm.

15. A unit according to at least one of claims 1 to 14 in which the parallel plates or tubes of the sedimentation separator have a surface roughness of less than Ra = 0.25 µm on their upward-facing surfaces or these surfaces are hydrophobically coated or they have a surface finish with a lotus flower effect.

16. A unit according to at least one of claims 1 to 15 in which the parallel plates or tubes of the sedimentation separator can be subjected to vibrations of a specific frequency and amplitude.

17. A unit according to claim 1, **characterized in that** the pre-culture fermenter (9) has a cross-section which is tapered in a downward direction, the stirrer of the pre-culture fermenter (23) is suspended in an eccentric fashion and the aeration of the pre-culture is carried out by means of a microsparging aeration unit (25).

18. A unit according to claim 1, **characterized in that** a large-area anchor stirrer (13) close to the membrane and an axially wound bubble-free aeration system (14) are used in the production fermenter.

19. A unit according to claim 18, wherein the stirrer blades of the anchor stirrer are tapered (13a) in the area close to the base (13a, 23a).

20. A unit according to at least one of claims 1 to 19, wherein an aeration unit (17) is additionally incorporated for aeration.

21. A unit according to claim 1, **characterized in that** a large-area blade stirrer (21) and inclined baffles (19) which are at a distance from the wall, from the fermenter base, and from the liquid surface, are arranged in the production fermenter.

22. A unit according to claim 1, **characterized in that** a large-area blade stirrer (21) is arranged eccentrically in the production fermenter.

23. A unit according to at least one of claims 1 to 22 , in which an aeration ring is additionally incorporated in the production fermenter for microsparging (17).

24. A unit according to at least one of claims 1 to 23 in which a hydrocyclone (11) or an ultrasonic separation system is arranged upstream of the sedimentation separator (4).

25. A unit according to at least one of claims 1 to 24 in which an agglomerate separator (12) is arranged downstream of the recycling outlet of the sedimentation separator (36, 47).

26. A unit according to at least one of claims 1 to 25 in which a throughflow heat exchanger (3) is arranged In each case between the outlet of the fermentation tank and the inlet of the sedimentation separator (34, 45, 48) and between the concentrate outlet of the sedimentation separator (36, 47) and the recycling inlet of the fermentation tank.

27. A unit according to at least one of claims 1 to 26 in which low-shear pumps (8) are applied which lead to a turbulent shear stress of less than or equal to 0.1 N/m².

28. A process for carrying out continuous high cell density fermentation, **characterized in that** a unit according to at least one of claims 1 to 28 is used.

29. A process according to claim 28 using a single-stage pre-culture step, **characterized in that** the pre-culture is carried out at least intermittently as a fed-batch process and the pre-culture volume Increases due to the fed batch to at least 3 to 6 times its starting volume.

30. A process according to claim 29, wherein the pre-culture is carried out at least intermittently as a continuous process with cell recycling.

31. A process according to claim 30 in which at the same time as the production culture a pre-culture is carried out as a continuous process with cell recycling.

## Patentansprüche

1. Einheit zum Durchführen der kontinuierlichen Fermentierung mit hoher Zelldichte, die einen Vorkulturfermenter (9), einen Substratvorratstank (1), einen Produktionsfermenter (2), einen Sedimentierungsseparator (4) und ein Abschöpfungsgefäß (6) umfasst, **dadurch gekennzeichnet, dass** der Sedimentierungsseparator eine Separatorfläche von Aₜₕ/Vₛ ≥30 m²/m³ auf der Basis des Separatorvolumens hat und der Sedimentierungsseparator eine konische oder pyramidenförmige Aufnahmekammer (32, 44) hat und der Zulauf in die Aufnahmekammer des Sedimentierungsseparators über mindestens zwei Rohrleitungen erfolgt, die radial (35), tangential in identischer Richtung (39, 34) oder tangential in entgegengesetzten Richtungen (41, 42) angeordnet sind, wobei die Rohrleitungen regelmäßig über dem Querschnitt angeordnet sind.

2. Einheit zum Durchführen der kontinuierlichen Fermentierung mit hoher Zelldichte nach Anspruch 1, **dadurch gekennzeichnet, dass** der Sedimentierungsseparator eine Separatorfläche von Aₜₕ ≥0,5 m² und gleichzeitig entweder eine spezifische Oberfläche von Aₜₕ/Vₛ ≥30 m²/m³ (auf der Basis des Separatorvolumens) oder eine spezifische Oberfläche von Aₜₕ/V ≥5 m²/m³ (auf der Grundlage des Fermentervolumens) hat, während die Einheit mit Perfusionsraten im Bereich von 5 bis 15 Fermentervolumina pro Tag betrieben werden kann.

3. Einheit zum Durchführen der kontinuierlichen Fermentierung mit hoher Zelldichte nach Anspruch 1 und 2, **dadurch gekennzeichnet, dass** der Sedimentierungsseparator eine Separatoroberfläche von Aₜₕ/Vₛ 50 - 100 m²/m³ auf der Grundlage des Separatorvolumens hat.

4. Einheit nach einem der Ansprüche 1 bis 3, wobei der Zulauf in den Sedimentierungsseparator (4) über die Rohrleitungen erfolgt, die tangential in identischer Richtung in einem ringförmigen Kanal (40) angeordnet sind, welcher sich außerhalb der Aufnahmekammer befindet.

5. Einheit nach mindestens einem der Ansprüche 1 bis 4, bei der der Zulauf (35) in die Aufnahmekammer des Sedimentierungsseparators (4) in einer geometrischen Höhe oberhalb der Grundplatte der Aufnahmekammer erfolgt, die größer als die Hälfte der Gesamthöhe der Aufnahmekammer und kleiner als das 0,8-Fache, insbesondere bevorzugt kleiner als das 0,75-Fache der Gesamthöhe der Aufnahmekammer ist.

6. Einheit nach mindestens einem der Ansprüche 1 bis 4, bei der der Sedimentierungsseparator gemäß dem Kreuzstromprinzip betrieben wird.

7. Einheit nach Anspruch 6, bei der ein zusätzlicher Zulaufkanal Fermentierungsmedium direkt in die Aufnahmekammer (33, 44) transportiert, wodurch die Verweilzeit der Zellen in der Aufnahmekammer reduziert wird.

8. Einheit nach mindestens einem der Ansprüche 1 bis 7, bei der die parallelen Platten oder Rohre des Sedimentierungsseparators innerhalb eines rechteckigen Moduls angeordnet sind, dessen Verhältnis von Querschnittshöhe zu Querschnittsbreite annähernd dem Sinus des Winkels α zwischen der Horizontalen und dem Neigungswinkel des Moduls in montiertem Zustand entspricht.

9. Einheit nach mindestens einem der Ansprüche 1 bis 8, bei der der Zulauf in den Sedimentierungsseparator über kreisförmige Diffusoren (35) erfolgt, die einen halben Kegelwinkel von höchstens 6° haben, oder über flache Diffusoren (39), die ein longitudinales Differential der Querschnittsfläche, die durch die Peripherie geteilt ist (1/P dA/ds), von 0,1 oder darunter bei Geschwindigkeiten von höchstens 0,1 m/s haben.

10. Einheit nach mindestens einem der Ansprüche 1 bis 9, wobei der Sedimentierungsseparator aus einem rechtwinkligen Modul (29) besteht, in dem die einzelnen Kanäle (31) räumlich voneinander durch Platten (30) getrennt sind und die Platten in Nuten im Modul geführt und gehalten werden und die Platten bei Bedarf montiert oder demontiert werden können.

11. Einheit nach mindestens einem der Ansprüche 1 bis 5 und 8 bis 10, bei der die geneigten rechtwinkligen Kanäle oder Rohre des Sedimentierungsseparators eine Länge von 50 cm oder mehr haben und die entsprechenden Kanalhöhen kleiner oder gleich 10 mm sind.

12. Einheit nach mindestens einem der Ansprüche 1 bis 5 und 8 bis 10, bei der die geneigten rechtwinkligen Kanäle oder Rohre des Sedimentierungsseparators eine Länge von 50 cm oder mehr haben und die Kanalhöhen 4 bis 6 mm sind.

13. Einheit nach mindestens einem der Ansprüche 6 bis 7, bei der der Kreuzstrom-Sedimentierungsseparator geneigte rechtwinklige Kanäle hat, die eine Länge von 20 cm oder mehr haben, und die Kanäle eine Höhe von 10 mm oder darunter haben.

14. Einheit nach mindestens einem der Ansprüche 6 bis 7, bei der der Kreuzstrom-Sedimentierungsseparator geneigte rechtwinklige Kanäle hat, die eine Länge von 20 cm oder mehr haben, und die Kanäle eine Höhe von 4 bis 6 mm haben.

15. Einheit nach mindestens einem der Ansprüche 1 bis 14, bei der die parallelen Platten oder Rohre des Sedimentierungsseparators eine Oberflächenrauheit von weniger als Ra = 0,25 µm auf den nach oben zeigenden Oberflächen haben oder diese Oberflächen hydrophob beschichtet sind oder sie eine Oberflächenqualität mit einem Lotusblüteneffekt haben.

16. Einheit nach mindestens einem der Ansprüche 1 bis 15, bei der die parallelen Platten oder Rohre des Sedimentierungsseparators Vibrationen einer bestimmten Frequenz und Amplitude ausgesetzt sein können.

17. Einheit nach Anspruch 1, **dadurch gekennzeichnet, dass** der Vorkulturfermenter (9) einen Querschnitt hat, der nach unten zu konisch ist, die Rührvorrichtung des Vorkulturfermenters (23) exzentrisch aufgehängt ist und die Belüftung der Vorkultur mittels einer Microsparging-(Mikrobelüftungs)-Einheit (25) erfolgt.

18. Einheit nach Anspruch 1, **dadurch gekennzeichnet, dass** eine großflächige Ankerrührvorrichtung (13) in der Nähe der Membran und ein axial gewundenes blasenfreies Belüftungssystem (14) im Produktionsfermenter verwendet werden.

19. Einheit nach Anspruch 18, wobei die Rührflügel des Ankerrührers im Bereich in der Nähe der Basis (13a, 23a) verjüngt sind (13a).

20. Einheit nach mindestens einem der Ansprüche 1 bis 19, wobei zusätzlich eine Belüftungseinheit (17) zur Belüftung integriert ist.

21. Einheit nach Anspruch 1, **dadurch gekennzeichnet, dass** eine großflächige Flügelrührvorrichtung (21) und geneigte Leitflächen (19), die von der Wand, von der Fermenterbasis und von der Flüssigkeitsoberfläche beabstandet sind, im Produktionsfermenter angeordnet sind.

22. Einheit nach Anspruch 1, **dadurch gekennzeichnet, dass** eine großflächige Flügelrührvorrichtung (21) exzentrisch im Produktionsfermenter angeordnet ist.

23. Einheit nach mindestens einem der Ansprüche 1 bis 22, bei der zusätzlich ein Belüftungsring in den Produktionsfermenter zur Mikrobelüftung (17) integriert ist.

24. Einheit nach mindestens einem der Ansprüche 1 bis 23, bei der ein Hydrozyklon (11) oder ein Ultraschalltrennsystem stromaufwärts des Sedimentierungsseparators (4) angeordnet ist.

25. Einheit nach mindestens einem der Ansprüche 1 bis 24, bei der ein Agglomeratabscheider (12) stromabwärts des Recyclingausgangs des Sedimentierungsseparators (36, 47) angeordnet ist.

26. Einheit nach mindestens einem der Ansprüche 1 bis 25, bei der ein Durchlaufwärmetauscher (3) in jedem Fall zwischen dem Ausgang des Fermentierungstanks und dem Eingang des Sedimentierungsseparators (34, 45, 48) und zwischen dem Konzentratausgang des Sedimentierungsseparators (36, 47) und dem Recyclingeingang des Fermentierungstanks angeordnet ist.

27. Einheit nach mindestens einem der Ansprüche 1 bis 26, bei der scherarme Pumpen (8) eingesetzt werden, die zu einer turbulenten Schubspannung von kleiner oder gleich 0,1 N/m² führen.

28. Prozess zum Durchführen einer kontinuierlichen Fermentierung mit hoher Zelldichte, **dadurch gekennzeichnet, dass** eine Einheit gemäß mindestens einem der Ansprüche 1 bis 28 verwendet wird.

29. Prozess nach Anspruch 28, der einen einstufigen Vorkulturschritt verwendet, **dadurch gekennzeichnet, dass** die Vorkultur zumindest intermittierend als Fed-Batch-Prozess ausgeführt ist und das Vorkulturvolumen sich auf Grund des Fed-Batch auf mindestens das 3- bis 6-Fache seines Anfangsvolumens erhöht.

30. Prozess nach Anspruch 29, wobei die Vorkultur zumindest intermittierend als kontinuierlicher Prozess mit Zellrecycling ausgeführt wird.

31. Prozess nach Anspruch 30, bei dem zur selben Zeit wie die Produktionskultur eine Vorkultur als kontinuierlicher Prozess mit Zellrecycling ausgeführt wird.

## Revendications

1. Unité destinée à exécuter une fermentation en continu à haute densité de cellules et comportant un fermenteur (9) de pré-culture, une cuve (1) de stockage de substrat, un fermenteur de production (2), un séparateur (4) à sédimentation et un récipient de collecte (6),
**caractérisée en ce que**
le séparateur à sédimentation présente une superficie de séparation Aₜₕ/Vₛ ≥ 30 m²/m³, par rapport au volume du séparateur et le séparateur à sédimentation présente une chambre conique ou pyramidale de réception (32, 44), l'entrée dans la chambre de réception du séparateur de sédimentation s'effectuant par l'intermédiaire d'au moins deux conduits agencés radialement (35) et tangentiellement dans la même direction (39, 34) ou tangentiellement dans des directions opposées (41, 42), les conduits étant agencés de manière régulière sur la section transversale.

2. Unité destinée à exécuter une fermentation en continu à haute densité de cellules selon la revendication 1, **caractérisée en ce que** le séparateur à sédimentation présente une superficie de séparation Aₜₕ ≥ 0,5 m² et en même temps une superficie spécifique Aₜₕ/Vₛ ≥ 30 m²/m³ (par rapport au volume du séparateur) ou une superficie spécifique Aₜₕ/V ≥ 5 m²/m³ (par rapport au volume du fermenteur), l'unité pouvant être conduite à des débits de traversée de l'ordre de 5 à 15 volumes de fermenteur par jour.

3. Unité destinée à exécuter une fermentation en continu à haute densité de cellules selon les revendications 1 et 2, **caractérisée en ce que** le séparateur à sédimentation présente une superficie de séparation Aₜₕ/Vₛ de 50 à 100 m²/m³ par rapport au volume du séparateur.

4. Unité selon l'une quelconque des revendications 1 à 3, dans laquelle l'entrée de l'écoulement dans le séparateur à sédimentation (4) a lieu par l'intermédiaire des conduits agencés tangentiellement dans la même direction dans un canal annulaire (40) situé à l'extérieur de la chambre de réception.

5. Unité selon au moins l'une des revendications 1 à 4, dans laquelle l'entrée (35) de l'écoulement dans la chambre de réception du séparateur (4) à sédimentation a lieu à une hauteur géométrique au-dessus de la base de la chambre de réception qui est supérieure à la moitié de la hauteur totale de la chambre de réception et inférieure à 0,8 fois et de façon plus préférable inférieure à 0,75 fois la hauteur totale de la chambre de réception.

6. Unité selon au moins l'une des revendications 1 à 4, dans laquelle le séparateur de sédimentation travaille selon le principe des écoulements croisés.

7. Unité selon la revendication 6, dans laquelle un canal supplémentaire d'entrée de l'écoulement transporte le fluide de fermentation directement dans la chambre de réception (33, 44) en réduisant ainsi le temps de séjour des cellules dans ladite chambre de réception.

8. Unité selon au moins l'une des revendications 1 à 7, dans laquelle les plaques ou tubes parallèles du séparateur à sédimentation sont agencés à l'intérieur d'un module rectangulaire dont le rapport entre la hauteur en coupe transversale et la largeur en coupe transversale correspond approximativement au sinus de l'angle α entre l'horizontale et l'angle d'inclinaison du module à l'état assemblé.

9. Unité selon au moins l'une des revendications 1 à 8, dans laquelle l'entrée de l'écoulement dans le séparateur à sédimentation a lieu par l'intermédiaire du diffuseur circulaire (35) dont le demi-angle de cône est d'au moins 6° ou par l'intermédiaire de diffuseurs plats (39) dont la différentielle longitudinale entre la superficie de la section transversale et la périphérie (1/P dA/ds) est de 0,1 ou moins, à des vitesses d'au plus 0,1 m/s.

10. Unité selon au moins une des revendications 1 à 9, dans laquelle ledit séparateur à sédimentation est constitué d'un module rectangulaire (29) dans lequel des canaux individuels (31) sont séparés spatialement l'un de l'autre par des plaques (30), lesdites plaques étant guidées et maintenues dans des rainures du module, lesdites plaques pouvant être montées ou démontées si nécessaire.

11. Unité selon au moins l'une des revendications 1 à 5 et 8 à 10, dans laquelle les canaux rectangulaires ou tubes inclinés du séparateur à sédimentation ont une longueur de 50 cm ou davantage, la hauteur correspondante des canaux étant inférieure ou égale à 10 mm.

12. Unité selon au moins l'une des revendications 1 à 5 et 8 à 10, dans laquelle les canaux rectangulaires ou tubes inclinés du séparateur à sédimentation ont une longueur de 50 cm ou davantage, la hauteur des canaux étant de 4 à 6 mm.

13. Unité selon au moins l'une des revendications 6 à 7, dans laquelle le séparateur de sédimentation à courants croisés présente des canaux rectangulaires inclinés d'une longueur de 20 cm ou davantage, les canaux ayant une hauteur de 10 mm ou moins.

14. Unité selon au moins l'une des revendications 6 à 7, dans laquelle le séparateur de sédimentation à courants croisés présente des canaux rectangulaires inclinés d'une longueur de 20 cm ou davantage, les canaux ayant une hauteur de 4 à 6 mm.

15. Unité selon au moins l'une des revendications 1 à 14, dans laquelle les plaques ou tubes parallèles du séparateur à sédimentation ont une rugosité de surface inférieure à Ra = 0,25 µm sur leur face tournée vers le haut, ces surfaces étant rendues hydrophobes par un revêtement ou ayant un fini de surface à effet de fleur de lotus.

16. Unité selon au moins l'une des revendications 1 à 15, dans laquelle les plaques ou tubes parallèles du séparateur à sédimentation peuvent subir des vibrations d'une fréquence et d'une amplitude spécifiques.

17. Unité selon la revendication 1, **caractérisée en ce que** le fermenteur (9) de pré-culture a une section transversale qui se rétrécit vers le bas, l'agitateur du fermenteur (23) de pré-culture étant suspendu en position décentrée et l'aération de la pré-culture étant conduite au moyen d'une unité d'aération (25) à micro-dispersion.

18. Unité selon la revendication 1, **caractérisée en ce qu'**un agitateur (13) à ancre de grande surface proche de la membrane et un système d'aération (14) sans bulles et enroulé axialement sont utilisés dans le fermenteur de production.

19. Unité selon la revendication 18, dans laquelle les pales de l'agitateur à ancre se rétrécissent (13a) dans la zone proche de la base (13a, 23a).

20. Unité selon au moins l'une des revendications 1 à 19, dans laquelle une unité d'aération (17) est de plus incorporée pour assurer l'aération.

21. Unité selon la revendication 1, **caractérisée en ce qu'**un agitateur (21) à pales de grande surface et des chicanes inclinées (19) situées à distance de la paroi, de la base du fermenteur et de la surface du liquide sont agencés dans le fermenteur de production.

22. Unité selon la revendication 1, **caractérisée en ce qu'**un agitateur (21) à pales de grande surface est agencé en position décentrée dans le fermenteur de production.

23. Unité selon au moins l'une des revendications 1 à 22, dans laquelle un anneau d'aération est de plus incorporé dans le fermenteur de production pour assurer la micro-dispersion (17).

24. Unité selon au moins l'une des revendications 1 à 23, dans laquelle un hydrocyclone (11) ou un système de séparation par ultrasons sont agencés en amont du séparateur (4) à sédimentation.

25. Unité selon au moins l'une des revendications 1 à 24, dans laquelle un séparateur (12) d'agglomérats est agencé en aval de la sortie de recyclage du séparateur à sédimentation (36, 47).

26. Unité selon au moins l'une des revendications 1 à 25, dans laquelle un échangeur de chaleur (3) traversé par un écoulement est agencé entre la sortie de la cuve de fermentation et l'entrée du séparateur à sédimentation (34, 45, 48) ainsi qu'entre la sortie de concentré du séparateur à sédimentation (36, 47) et l'entrée de recyclage de la cuve de fermentation.

27. Unité selon au moins l'une des revendications 1 à 26, dans laquelle des pompes (8) à bas cisaillement sont utilisées et conduisent à une contrainte de cisaillement turbulent inférieure ou égale à 0,1 N/m².

28. Procédé de conduite d'une fermentation en continu à haute densité de cellules, **caractérisé en ce qu'**il utilise une unité selon au moins l'une des revendications 1 à 28.

29. Procédé selon la revendication 28, utilisant une étape de pré-culture à un étage, **caractérisé en ce que** la pré-culture est conduite au moins par intermittence sous la forme d'une opération discontinue et **en ce que** le volume de pré-culture augmente suite à l'alimentation jusqu'à au moins 3 à 6 fois sont volume initial.

30. Procédé selon la revendication 29, dans lequel la pré-culture est conduite au moins par intermittence sous la forme d'une opération continue avec recyclage des cellules.

31. Procédé selon la revendication 30, dans lequel une pré-culture est conduite sous la forme d'une opération continue avec recyclage des cellules en même temps que la culture de production.
